# EUROPEAN PATENT SPECIFICATION

(11) **EP 3 990 917 B1**
(45) Date of publication and mention of the grant of the patent: **26.03.2025**
(21) Application number: 20831412.0
(22) Date of filing: 25.06.2020
(51) Int. Cl.: G01N 21/64, G01N 33/15

(54) **ANALYSIS OF OSCILLATORY FLUORESCENCE FROM BIOLOGICAL CELLS**
ANALYSE VON OSZILLIERENDER FLUORESZENZ AUS BIOLOGISCHEN ZELLEN
ANALYSE DE FLUORESCENCE OSCILLATOIRE PROVENANT DE CELLULES BIOLOGIQUES

(30) Priority: 25.06.2019 US 201962866524 P
(43) Date of publication of application: 04.05.2022
(73) Proprietor: Molecular Devices, LLC, San Jose, CA 95134 (US)
(72) Inventor: SIRENKO, Oksana, San Jose, CA 95134 (US); CRITTENDEN, Carole, San Jose, CA 95134 (US); SRIDHAR, Krithika, San Jose, CA 95134 (US); BREZDEN, Borys, San Jose, CA 95134 (US)
(74) Representative: Mollekopf, Gerd Willi
(86) International application number: PCT/US2020/039599
(87) International publication number: WO 2020/264148

(56) References cited:
- WO-A1-2006/069188
- US-A1- 2007 276 611
- US-A1- 2007 276 611
- US-B1- 7 038 106
- US-B2- 8 709 804
- SIRENKO OKSANA ET AL: "Multiparameter In Vivo Assessment of Compound Effects on Cardiomyocyte Physiology Using iPSC Cells", JOURNAL OF BIOMOLECULAR SCREENING, vol. 18, no. 1, 8 July 2012 (2012-07-08), pages 39 - 53, XP055773216, DOI: 10.1177/1087057112457590
- OKSANA SIRENKO , CAROLE CRITTENDEN , NICK CALLAMARAS , JAYNE HESLEY , YEN-WEN CHEN , CARLOS FUNES , IVAN RUSYN , BLAKE ANSON , EVA: "Multiparameter In Vivo Assessment of Compound Effects on Cardiomyocyte Physiology Using iPSC Cells", JOURNAL OF BIOMOLECULAR SCREENING, vol. 18, no. 1, 8 July 2012 (2012-07-08), pages 39 - 53, XP055773216, DOI: 10.1177/1087057112457590
- PRADA JUAN, SASI MANJU, MARTIN CORINNA, JABLONKA SIBYLLE, DANDEKAR THOMAS, BLUM ROBERT: "An open source tool for automatic spatiotemporal assessment of calcium transients and local ‘signal-close-to-noise’ activity in calcium imaging data", PLOS COMPUTATIONAL BIOLOGY, vol. 14, no. 13, e1006054, 30 March 2018 (2018-03-30), pages 1 - 34, XP055773223, DOI: 10.1371/journal.pcbi.1006054

## Description

### Field

The present disclosure relates to methods and systems for analyzing oscillatory fluorescence from biological cells. More specifically, the disclosed examples relate to methods and systems for analyzing oscillatory fluorescence representing an oscillating ion flux, such as calcium oscillations, in the biological cells, and for testing the effect of cell treatments on the oscillatory fluorescence.

### Introduction

A large percentage of drugs fail in clinical studies due to cardiac or neural toxicity. To reduce the incidence of these failures, sensitive in vitro assays are needed to reliably evaluate adverse effects of compounds on cardiac and neural cells before clinical studies begin. These in vitro assays could accelerate and streamline drug development.

Culture systems for cardiomyocytes and neurons have been developed in which these cells exhibit spontaneous, synchronized ion fluxes, such as calcium oscillations. The calcium oscillations can be detected by labeling the cells with a fluorescent calcium indicator.

Imaging systems including analysis software also have been developed to record and analyze oscillatory fluorescence representing calcium oscillations in cells. The imaging systems have been utilized to measure the impact of pharmacologic compounds on calcium oscillations of cardiomyocytes and neurons in culture. Significantly, these calcium oscillations are perturbed in vitro by pharmacologic compounds having known cardiotoxic or neurotoxic effects. Accordingly, this methodology shows promise for safety testing of drugs and other chemicals, and for predicting efficacy and dosing of drug candidates prior to clinical studies.

The oscillation patterns produced when calcium oscillations are perturbed by drugs and other compounds can be very complex. Better methods and systems are needed for detecting biologically-relevant features of these complex oscillation patterns, and extracting values for the most informative readouts.

"Multiparameter in Vitro Assessment of Compound Effects on Cardiomyocyte Physiology Using iPSC Cells" (Sirenko Oksana et al., Journal of Biomolecular Screening, vol. 18, no. 1, 8. July 2012) discloses techniques for measuring the impact of pharmacologic compounds on the beating rate of cardiomyocytes with ImageXpress Micro and FLIPR Tetra systems. The assays employ calcium-sensitive dyes to monitor changes in Ca2+ fluxes synchronous with cell beating, which allows monitoring of the beat rate, amplitude, and other parameters.

US 8 709 804 B2 discloses instrumentation and methods based on imaging and measuring single cell dose response by fluorescent ion imaging that records live cell responses to drug doses. Dose response curves and other pharmacological parameters can be computed by imaging and measuring oscillation changes for each drug dose and each cell.

US 2007/0276611 A1 discloses an electrochemical sensor in which a peak is detected by grouping data points in windows and detecting two or three windows between which the slope of the data changes sign. The peak can be more precisely detected by detecting the highest data point in the two or three windows.

### Summary

The invention is defined in the independent claims. Particular embodiments are set out in the dependent claims.

The present disclosure provides methods and systems for recording and analyzing oscillatory fluorescence representing an oscillatory ion flux associated with one or more biological cells. An illustrative method of analysis may comprise detecting fluorescence from one or more biological cells to produce a series of data points describing an oscillation pattern. A series of slopes may be calculated for the oscillation pattern. A sliding window may be used to define subsets of the series of data points from which the series of slopes are calculated. Peaks of the oscillation pattern may be identified using the series of slopes. A method of analysis comprises detecting fluorescence from one or more biological cells to produce a series of data points describing an oscillation pattern. Primary peaks and secondary peaks, if any, in the oscillation pattern are identified. An aspect of the secondary peaks is determined.

### Brief Description of the Drawings

Figure 1 is a flowchart of steps that may be performed in an illustrative method of analyzing oscillatory fluorescence patterns from biological cells.
Figure 2 is a schematic view of an illustrative system for detecting and analyzing oscillatory fluorescence patterns from biological cells.
Figure 3 is a graph of a relatively simple, unperturbed oscillation pattern produced by a fluorescence signal detected from biological cells undergoing oscillatory ion flux.
Figure 4 is a graph of a series of data points for a region of the fluorescence signal of Figure 3 encompassing a single oscillation, with the region indicated generally at "4" in Figure 3, and with a sliding window shown schematically at three different temporal positions to illustrate how subsets of the data points can be selected with the sliding window for calculation of a series of slopes.
Figure 5 is a graph of a single oscillation (an "event") of a more complex, perturbed oscillation pattern detected as in Figure 3, and illustrating how slopes calculated as in Figure 4 can be used to detect biologically relevant peaks and troughs, while ignoring smaller "spurious" fluctuations in the fluorescence signal that are assumed to be noise.
Figure 6 is a graph of only a portion of the single oscillation of Figure 5 taken around a secondary peak and illustrating how primary/secondary peaks can be filtered using predefined amplitude and/or duration criteria to reject small and/or transient peaks as invalid.
Figure 7 is a graph of an oscillation pattern detected as in Figure 3 and illustrating aspects of an algorithm for automatically setting a baseline for the oscillation pattern, namely, setting a temporary reference line and a threshold line relative to the reference line.
Figure 8 is a fragmentary view of a bottom portion of the oscillation pattern of Figure 7 showing negative peaks (with respect to the reference line of Figure 7) located below the threshold line and identified based on slopes.
Figure 9 is a graph of three oscillations of a complex oscillation pattern detected generally as in Figure 3, with illustrative parameters identified.
Figures 10 and 11 are graphs of the same complex oscillation pattern analyzed using different sizes for the sliding window (eleven points and five points, respectively), with primary peaks marked by circles and secondary peaks by diamonds.
Figures 12A and 12B are fragmentary views of an exemplary dialog box of a graphical user interface created by analysis software, with an "Options" tab of the dialog box selected.
Figures 13A and 13B are fragmentary views of the dialog box of Figures 12A and 12B, except with a "Measurements" tab of the dialog box selected.
Figures 14-21 are graphs of representative fluorescence oscillation patterns detected from cardiomyocytes treated as a control (Figure 14) and with various known cardiotoxic compounds (Figures 15-21). Primary ("main") peaks and secondary peaks are marked by software with circles and diamonds, respectively.
Figure 22 is a graph plotting various peak-related readouts obtained with software as disclosed herein, by analyzing fluorescence oscillation patterns detected from cardiomyocytes treated with the indicated high risk, medium risk, and low risk TdP (torsades de pointes) compounds, at the indicated concentrations relative to a maximum clinical level in blood for each compound.
Figures 23-30 are graphs of representative fluorescence oscillation patterns detected from neurons treated as a control (Figure 23) and with various known neurotoxic compounds (Figures 24-30). Primary peaks and secondary peaks are marked by software with circles and diamonds, respectively. Modifications in oscillation patterns reflect effects of the compounds and can be characterized via multiple measurements provided by software analysis.

### Detailed Description

The present disclosure provides methods and systems for recording and analyzing oscillatory fluorescence representing an oscillatory ion flux associated with one or more biological cells. An illustrative method of analysis may comprise detecting fluorescence from one or more biological cells to produce a series of data points describing an oscillation pattern (interchangeably called an oscillation trace or fluorescence trace). A series of slopes may be calculated for the oscillation pattern. For example, a sliding window may be used to define subsets of the series of data points from which the series of slopes are calculated. Peaks of the oscillation pattern may be identified using the series of slopes. Another illustrative method of analysis may comprise detecting fluorescence from one or more biological cells to produce a series of data points describing an oscillation pattern. Primary peaks and secondary peaks, if any, in the oscillation pattern may be identified. An aspect of the secondary peaks may be determined.

The methods and systems of the present disclosure may utilize a complex event analysis (CEA) algorithm designed to analyze oscillatory fluorescence having several components of interest. These components may include multiple shapes of peaks, clustering of peaks, secondary peaks, regular or irregular anomalous events, irregular amplitudes or frequencies, migrating threshold levels, etc. The CEA algorithm has the ability to report multiple measurements for both primary and secondary peaks, either averaged or individual values, including many additional readouts.

The objective of the CEA algorithm is to digitally "visualize" the general shape of biological events in order to detect and measure various parameters, such as peak amplitude, rise and decay times, event duration, frequency, etc. To visualize the biological events, spurious, non-biological transitions (noise) need to be rejected. Many "point-by-point" detection methods lose accuracy because of prior filtering, which compromises data integrity, or due to a failure to distinguish biologically relevant transitions from noise. By the nature of its design, the CEA algorithm significantly reduces the influence of noise, without prior filtering, by calculating slopes from subsets of data points describing an oscillation pattern. The slopes enable detection of peaks and troughs, while ignoring lower amplitude and/or rapid transitions (i.e., invalid peaks) generated by noise.

Further aspects of the present disclosure are described in the following sections: (I) definitions, (II) method and system overview, and (III) examples.

### I. Definitions

Technical terms used in this disclosure have meanings that are commonly recognized by those skilled in the art. However, the following terms may be further defined as follows.

Event - a single oscillation of an oscillation pattern. An event may begin and end at a predefined amplitude(s) from a baseline and/or within a predefined amplitude range(s) from the baseline, among others.

Light - ultraviolet, visible, and/or infrared radiation.

Maximum - a point of an actual/conceptual graph (and/or of a series of points) having a greater value than points around it, and/or being farther from a reference line (e.g., a bottom or top baseline) than points around it. The plural of "maximum" is "maxima."

Minimum - a point of an actual/conceptual graph (and/or of a series of points) having a lesser value than points around it, and/or being closer to a reference line (e.g., a bottom or top baseline) than points around it. The plural of "minimum" is "minima."

Peak - a sequence of points of an actual/conceptual graph (and/or of a series of points) including a maximum and points around the maximum, and optionally bounded by a pair of minima. A peak may be characterized according to the temporal position and the amplitude of the peak's maximum, which collectively may define a "peak position," while the amplitude of the maximum defines a "peak value" or "peak amplitude." When maxima are defined with respect to a reference line (e.g., a bottom or top baseline), maxima above the reference line form positive peaks, while maxima below the reference line form negative peaks.

Primary peak - the leading/only peak (or valid peak) of an event.

Secondary peak - any peak (or valid peak) following the primary peak within an event.

Trough - a sequence of points of an actual/conceptual graph (and/or of a series of points) including a minimum and points around the minimum. The trough may be characterized according to the temporal position and the amplitude of the trough's minimum, which may collectively define a "trough position," while the amplitude of the minimum defines a "trough value" or "trough amplitude." When minima are defined with respect to a reference line (such as a baseline), minima above the reference line form positive troughs, which minima below the reference line form negative troughs.

Valid peak - any peak meeting predefined criteria for validity.

Window - an algorithm that selects a given number of data points from a data set for processing. For example, a window having a width of five selects five points, such as five successive points of the data set, for processing. A "sliding window" moves across a data set, such as point-by-point, to select subsets of data points for processing. For example, a sliding window having a width of five points may process points 1-5, and then move point-by-point, to process points 2-6, 3-7, etc.

### II. Method and System Overview

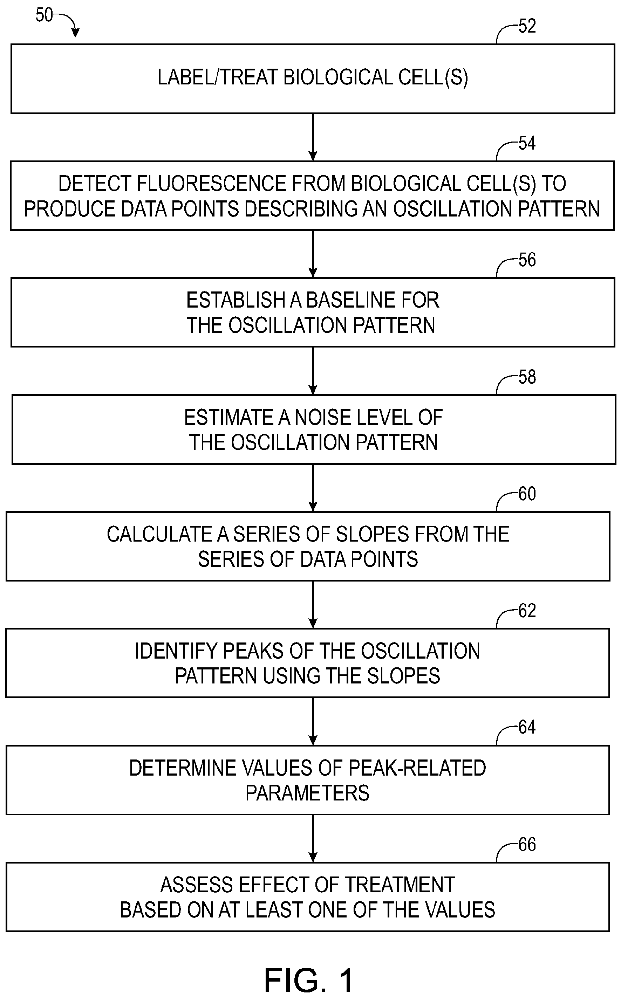

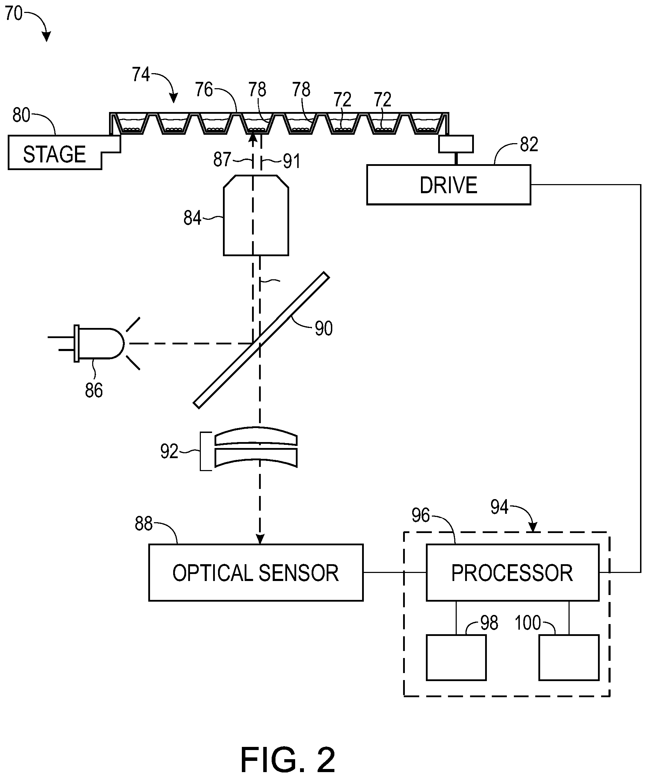

This section provides an overview of exemplary methods and systems of the present disclosure, see Figures 1 and 2.

Figure 1 shows a flowchart 50 of steps that may be performed in an illustrative method of analyzing oscillatory fluorescence from one or more biological cells (also called "cells"). The steps may be performed in any suitable order and combination.

The cells may include any cell type that exhibits oscillatory ion flux in culture. Accordingly, the cells may include muscle cells (i.e., cardiac muscle cells (cardiomyocytes), skeletal muscle cells, or smooth muscle cells) or nerve cells (neurons). The oscillatory ion flux may occur in a synchronized fashion, optionally spontaneously, for a set of the cells cultured in close association with one another, such that the cells can communicate. The set of cells may by associated with one another in a three-dimensional aggregate, such as a cell spheroid, or may be arranged as a substantial monolayer, among others. A single set of cells may be analyzed in a vessel, or isolated replicate sets of cells may be exposed to different treatments in separate vessels before/during analysis, as explained further below. Exemplary vessels include petri dishes, wells of a microplate(s), flasks, or the like.

The cells may be obtained from any suitable source(s) by any suitable procedures. The cells may be differentiated in vitro from stem cells. The stem cells may be embryonic stem cells, adult stem cells, or induced pluripotent stem cells (iPSC), among others. In other examples, the cells may be primary cells, such as primary cardiomyocytes or primary neurons obtained from an animal.

The oscillatory ion flux may be an ion-specific flux or a collective ion flux. An exemplary ion-specific flux is an oscillatory calcium flux that produces calcium oscillations. The flux may represent movement of ions across the plasma membrane (i.e., into and/or out of cells), and/or within cells (e.g., across the membrane of the sarcoplasmic reticulum (SR) (i.e., into or out of the cytoplasm from the SR), or the like.

Each set of cells may be labeled and/or treated, indicated at 52. Labeling may be performed with a fluorescent indicator having a fluorescence that is sensitive to an oscillatory ion flux occurring with respect to the cells. The fluorescent indicator may, for example, be a fluorescent calcium indicator that is sensitive to the intracellular calcium concentration and emits more light (or less light) as the intracellular calcium concentration increases. Exemplary fluorescent calcium indicators are chemical indicators, such as FLIPR^{®} Calcium 6, FLIPR^{®} Calcium 6-QF, Calcium Green-1, Fluo-3, Fluo-4, Fura-2, Indo-1, Oregon Green 488, Bapta-1, Fura-4F, Fura-5F, Calcium Crimson, X-rhod-1, and the like. The cells may be labeled with a chemical indicator by contact via the medium in which the cells are disposed. Other exemplary fluorescent calcium indicators are genetically-encoded and expressed in the cells, after introduction of a coding sequence into the cells (such as via transfection, infection, or the like). Suitable genetically-encoded calcium indicators may include Cameleons, Pericams, GCaMP, TN-L15, TN-humTnC, TN-XL, TN-XXL, Twitches, or the like. In other examples, the fluorescent indicator may be a membrane potential indicator (e.g., FluoVolt^{®} Membrane Potential Dye, Di-3-ANEPPDHQ, Di-4-ANEPPDHQ, etc.), a potassium indicator, a sodium indicator, a magnesium indicator, a zinc indicator, a pH indicator, or the like.

Each set of cells is treated with at least one substance of interest at a selected concentration, to test the substance/concentration for an effect, if any, on an oscillatory fluorescence of the cells detected from the fluorescent indicator. The substance may be a compound, such as a small molecule (e.g., a drug or drug candidate), having a molecular weight of less than 10, 5, 2, or 1 kilodaltons, among others), a protein, an RNA or DNA molecule, etc. Different compounds and/or different concentrations of the same compound may be tested on respective sets of the cells to screen compounds and/or determine dose-response curves for a given compound. Each set of cells may be treated with a substance for any suitable length of time, such as at least 1, 2, 5, 10, 30, or 60 minutes, or 2, 4, 6, 8, 10, 12, 18, or 24 hours, or 24-72 hours, among others. Treatment and labeling may be performed in parallel, serially, or at overlapping times with a temporal offset.

Fluorescence may be detected from at least one cell of each set of biological cells, indicated at 54. This fluorescence detection samples a fluorescence signal from the at least one cell, such as the signal's intensity, with respect to time to produce a series of data points (also called time points) describing an oscillation pattern for the fluorescence signal. Accordingly, the oscillation pattern may be graphed as the detected fluorescence intensity as a function of time. The fluorescence signal may be sampled at any suitable rate, generally a constant rate of greater than 1 Hz, such as a rate of 1-100 Hz, among others, and for any suitable sampling duration, such as at least 10, 30, or 60 seconds, or at least 2, 5, or 10 minutes. Fluorescence detection may be performed with an image sensor, an optical point sensor, or the like.

A baseline for the oscillation pattern may be established, indicated at 56. The baseline may be set automatically using software, for example, as described below in Example 2, and/or may be set by a user via a graphical user interface. The baseline may be positioned at the bottom or the top of the oscillation pattern, either by default or in response to an input from a user (e.g., see Examples 2 and 5). In some embodiments, a temporary reference line may be set at the top (or bottom) of the oscillation pattern, opposite the prospective location of the baseline to be established. Peaks with respect to the reference line may be identified, and linear regression of peak or trough positions may find a line that serves as the baseline during subsequent identification of peaks and troughs.

A noise level of the oscillation pattern may be estimated, indicated at 58. The noise level may be estimated based on signal fluctuation near the top or the bottom of the oscillation pattern (see Examples 2 and 5). For example, a mean of the successive differences between peak values (i.e., maxima) (or trough values (i.e., minima)) near the baseline of the oscillation pattern may be used to determine an estimate of the noise level.

A series of slopes may be calculated from the series of data points of the oscillation pattern, indicated at 60. For example, a sliding window that slides along the time axis of the oscillation pattern may be utilized to select successive subsets of the data points for calculation of the series of slopes by fitting a line to each subset by linear regression (e.g., see Example 1). In other words, the sliding window may slide along the time axis of the oscillation pattern by one point (or two or more points) for calculation of each successive slope. The sliding window has a constant size that encompasses a fixed number of three or more points while the series of slopes are calculated for a given data set (e.g., from a given well). The size of the sliding window may be selected automatically by software (e.g., based on the noise level of the data set and/or the sampling rate of the data points), or may be selected by a user. The size of the sliding window may be changed between different data sets (e.g., with each data set collected from a different well), which allows the size to be optimized for each data set.

Peaks (and troughs) of each oscillation pattern may be identified using the slopes, indicated at 62. To wit, a series of maxima and minima may be identified based on changes in the sign of the slope along the series of slopes (i.e., with respect to time). Each maximum above a baseline can be closely approximated by a change within the series of slopes from positive to negative (or from negative to positive for a baseline at the top of the oscillation pattern). Similarly, each minimum above a baseline can be closely approximated by a change within the series of slopes from negative to positive (or from positive to negative for a baseline at the top of the oscillation pattern). The maxima and minima identified using the series of slopes may be refined if needed, by local analysis of points and/or interpolation, among others. The maxima and/or the minima (and the corresponding peaks and troughs) also may be filtered (i.e., restricted) according to one or more other predefined criteria, to reject maxima (and peaks) and/or minima (and troughs) that do not meet the predefined criteria. The predefined criteria may relate to at least one amplitude and/or duration of each corresponding peak/trough, and/or of each event containing at least one of the peaks (e.g., see Examples 1 and 5). The predefined criteria may be auto-assigned based on the noise level of the fluorescence signal, and/or may be assigned by a user subjectively.

Values of one or more peak-related parameters of each oscillation pattern may be determined, indicated at 64. The parameters and their values interchangeably may be called readouts or descriptors. The values may be determined using the peaks identified at 62. The values may be mean values for the oscillation pattern measurements (or at least for a plurality of events thereof), or values for individual events/peaks. Exemplary values are for primary peaks only, secondary peaks only, or primary and secondary peaks collectively. The values may include individual/mean primary/secondary peak amplitude(s), mean primary/secondary peak frequency, mean intra-event frequency of secondary peaks, event area, primary/secondary peak spacing, rise slope, decay slope, rise time, decay time, etc. Further examples of peak-related parameters are described in Examples 1, 3, and 5.

An effect, if any, of each treatment is assessed based on at least one of the values determined from the corresponding oscillation pattern, indicated at 66. The effect is assessed based on an aspect of the secondary peaks in the oscillation pattern, such as a number, frequency, and/or period of the secondary peaks.

Any combination of the steps of Figure 1, or other methods disclosed herein, may be embodied as a computer method, computer system, or computer program product. Accordingly, aspects of the analysis method may take the form of an entirely hardware example, an entirely software example (including firmware, resident software, micro-code, and the like), or an example combining software and hardware aspects, all of which may generally be referred to herein as a "circuit," "module," or "system." Furthermore, aspects of the analysis method may take the form of a computer program product embodied in a computer-readable medium (or media) having computer-readable program code/instructions embodied thereon.

Any combination of computer-readable media may be utilized. Computer-readable media can be a computer-readable signal medium and/or a computer-readable storage medium. A computer-readable storage medium may include an electronic, magnetic, optical, electromagnetic, infrared, and/or semiconductor system, apparatus, or device, or any suitable combination of these. More specific examples of a computer-readable storage medium may include the following: a portable computer diskette, a hard disk, a random-access memory (RAM), a read-only memory (ROM), an erasable programmable read-only memory (EPROM or Flash memory), a portable compact disc read-only memory (CD-ROM), an optical storage device, a magnetic storage device, any suitable combination of these, and/or the like. In the context of this disclosure, a computer-readable storage medium may include any suitable nontransitory, tangible medium that can contain or store a program for use by or in connection with an instruction execution system, apparatus, or device.

A computer-readable signal medium may include a propagated data signal with computer-readable program code embodied therein, for example, in baseband or as part of a carrier wave. Such a propagated signal may take any of a variety of forms, including, but not limited to, electromagnetic, optical, and/or any suitable combination thereof. A computer-readable signal medium may include any computer-readable medium that is not a computer-readable storage medium and that is capable of communicating, propagating, or transporting a program for use by or in connection with an instruction execution system, apparatus, or device.

Program code embodied on a computer-readable medium may be transmitted using any appropriate medium, including but not limited to wireless, wireline, optical fiber cable, and/or any suitable combination of these, among others.

Computer program code for carrying out operations for aspects of the methods disclosed herein may be written in one or any combination of programming languages, including an object-oriented programming language such as Java, Smalltalk, C++, and/or the like, and conventional procedural programming languages, such as C. Mobile apps may be developed using any suitable language, including those previously mentioned, as well as Objective-C, Swift, C#, HTML5, and the like. The program code may execute entirely on a user's computer, partly on the user's computer, as a stand-alone software package, partly on the user's computer and partly on a remote computer, or entirely on the remote computer or server. In the latter scenario, the remote computer may be connected to the user's computer through any type of network, including a local area network (LAN) or a wide area network (WAN), and/or the connection may be made to an external computer (for example, through the Internet using an Internet Service Provider).

Aspects of the methods disclosed herein are described below with reference to flowchart illustrations and/or block diagrams of methods, apparatus, systems, and/or computer program products. Each block and/or combination of blocks in a flowchart and/or block diagram may be implemented by computer program instructions. The computer program instructions may be provided to a processor of a general-purpose computer, special purpose computer, or other programmable data processing apparatus to produce a machine, such that the instructions, which execute via the processor of the computer or other programmable data processing apparatus, implement the functions/acts specified in the flowchart and/or block diagram block(s). In some examples, machine-readable instructions may be programmed onto a programmable logic device, such as a field programmable gate array (FPGA).

The computer program instructions can also be stored in a computer-readable medium that can direct a computer to function in a particular manner, such that the instructions stored in the computer-readable medium produce an article of manufacture including instructions which implement the functions/acts specified in the flowchart and/or block diagram block(s).

The computer program instructions can also be loaded onto a computer to cause a series of operational steps to be performed on the device to produce a computer-implemented process such that the instructions which execute on the computer provide processes for implementing the functions/acts specified in the flowchart and/or block diagram block(s).

Any flowchart and/or block diagram in the drawings is intended to illustrate the architecture, functionality, and/or operation of possible implementations of systems, methods, and computer program products according to aspects of the methods disclosed herein. In this regard, each block may represent a module, segment, or portion of code, which comprises one or more executable instructions for implementing the specified logical function(s). In some implementations, the functions noted in the block may occur out of the order noted in the drawings. For example, two blocks shown in succession may, in fact, be executed substantially concurrently, or the blocks may sometimes be executed in the reverse order, depending upon the functionality involved. Each block and/or combination of blocks may be implemented by special purpose hardware-based systems (or combinations of special purpose hardware and computer instructions) that perform the specified functions or acts.

Figure 2 shows an illustrative system 70 for detecting and analyzing oscillatory fluorescence from biological cells 72 held by a sample holder 74. Sample holder 74 is depicted here as a microplate 76 having a plurality of wells 78 each containing a replicate set of cells 72. System 70 may include a stage 80 to support the sample holder, and a drive mechanism 82 to move stage 80 and sample holder 74 relative to one another to place each well 78 on the optical axis of system 70.

System 70 also may comprise an objective 84, a light source 86, and an optical sensor 88 (e.g., an image sensor). Light source 86 may generate light 87 to irradiate each set of cells 72, to induce fluorescence from the cells. The light for cell irradiation may propagate to the cells via a beam-splitter 90 and objective 84, in the epi-illumination configuration shown in Figure 2. Fluorescence 91 induced by the light may be collected by objective 84 for propagation through objective 84, beam-splitter 90, and an optional tube lens 92, for detection by optical sensor 88, to create a time-dependent fluorescence signal.

A computer 94 is in communication with optical sensor 88, and processes the fluorescence signal received from optical sensor 88. Computer 94 may include a processor 96 to process instructions, memory 98 to store the instructions, and a user interface 100 for communication between computer 94 and a user. The user interface may include a user input device, such as a keyboard, mouse, or touchscreen, and a display device, such as a monitor.

### III. Examples

This section describes further examples and aspects of the analysis methods and systems of the present disclosure. These examples and aspects are intended for illustration only and should not limit the entire scope of the invention.

### Example 1. Calculation of Slopes

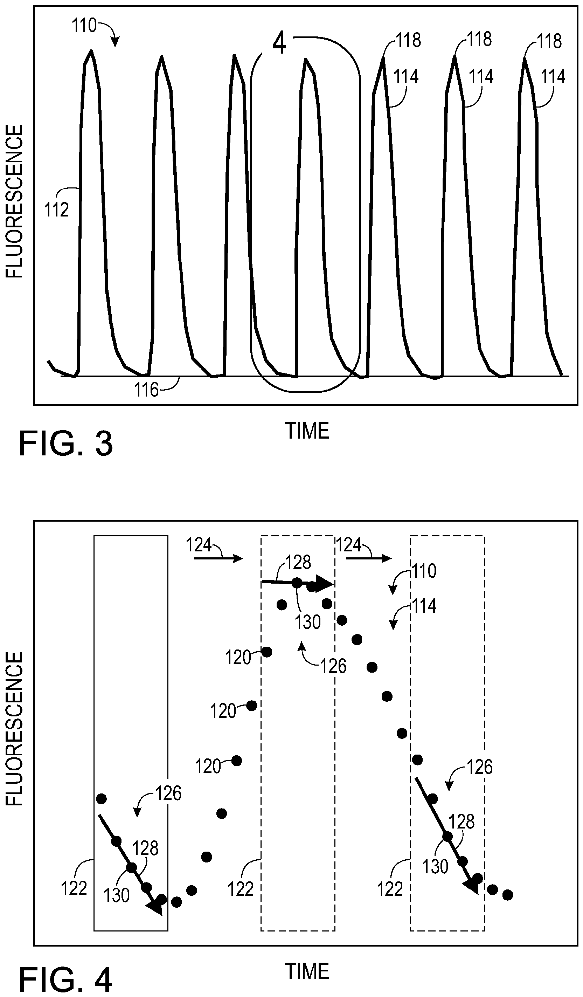

This example describes exemplary calculation of slopes for an oscillation pattern using a sliding window, and use of the slopes to identify peaks and troughs in the oscillation pattern; see Figures 3-6.

Figure 3 shows a graph of an oscillation pattern 110 produced by a fluorescence signal 112 detected from biological cells undergoing a simple oscillatory flux of calcium. Fluorescence is detected from a fluorescent calcium indicator that labels the cells. The fluorescence intensity is detected at discrete times, to produce a series of data points, and the data points are plotted as a function of time using a point-connecting line trace.

Oscillation pattern 110 is composed of a series of oscillations 114 (also called events) of increased fluorescence from a baseline 116. Each oscillation 114 has only one peak 118, as shown here, or has a primary peak and one or more secondary peaks, as described below. In other examples, baseline 116 may be positioned at the top of oscillation pattern 110, and each event may be characterized by the fluorescence signal 112 traveling below the baseline.

Figure 4 shows a graph of a sub-series of data points 120 from oscillation pattern 110 of Figure 3 for a single oscillation 114 of the fluorescence signal. The point-connecting line trace of Figure 3 is omitted. A sliding window 122 may be used to select subsets of data points 120 from which a series of best-fit lines and a corresponding series of slopes, optionally centered at each data point, may be computed. The size of sliding window 122 represents a width thereof measured parallel to the time axis. The sliding window 122 is shown schematically on the left in solid outline, and in dashed outline in two other illustrative positions, after conceptually sliding along the time axis, indicated by motion arrows at 124. Sliding window 122 "stops" at a plurality of incremental positions along the time axis, and selects a subset 126 of data points 120 centered at each position from which a slope is calculated. Here, the sliding window 122 has a width that selects five data points 120 at each incremental position, but any suitable number of three or more data points 120 may be selected. The size (i.e., the duration) of the sliding window may remain constant, to select the same number of data points 120 for calculation of each slope, while a series of slopes is calculated for a given oscillation pattern 110 (i.e., data set of data points). A slope may be calculated for each point. The size of the sliding window may be changed to re-calculate a new series of slopes for the same oscillation pattern, or to calculate a series of slopes for a different oscillation pattern. The series of slopes calculated for an oscillation pattern may represent a corresponding series of incrementally offset positions of sliding window 122. In other words, sliding window 122 may be successively offset incrementally by a fixed, integral number of data points, such as one or two data points, among others, and/or by a fixed time increment, to select subsets 126 of data points 120. Subsets 126 of data points 120 may overlap one another, if the size of sliding window 122 is greater than the incremental offset of the sliding window for successive subsets.

In some embodiments, a slope is calculated at each data point, and the "sliding window" at which the slope is computed moves one point at a time. In these embodiments, the subsets of data points selected by the window invariably overlap one another. The "direction" of the slope at each point is evaluated to assess the overall curvature of the event as implied by changes in the direction of the slopes. The slope calculated at each point is both forward-looking and backward-looking because one or more points temporally ahead of the point and one or more points temporally behind the point contribute to the slope.

A slope for each subset 126 may be calculated by fitting a straight line to points 120 of the subset 126 by linear regression, and then taking the slope of the line. Figure 4 shows three vectors 128 that are parallel to the respective lines fitted to three illustrative subsets 126. The orientation of each vector 128 matches the slope of the corresponding fitted line. Each vector 128 may be centered on the middle point(s) 130 of the corresponding subset 126 and may have a time-component that matches the size (i.e., the width) of sliding window 122. Accordingly, a series of vectors 128 can be generated with sliding window 122 to associate the size of the sliding window with a series of slopes having a corresponding series of data points 130 or positions (time and amplitude).

The series of slopes of vectors 128 enables examining the shape of the data, and particularly the direction of data excursion. For a baseline located at the bottom of the oscillation pattern, a positive slope reflects a rising phase while a negative slope is associated with a decay phase. Because each slope is calculated from a subset 126 having three or more points 120, noise within the subset 126 does not significantly affect the accuracy of the slope, if the noise level is low relative to the size of the sliding window. In this manner, noise is inherently rejected. Changes in the direction of successive vectors 128 reflect corresponding directional changes of transitions within an oscillation pattern. Thus, when the slope of successive vectors 128 changes from positive to negative (for a baseline at the bottom of the oscillation pattern), it is an indication that a maximum of a peak has been traversed. Similarly, with the same baseline, when the slope changes from negative to positive, it is an indication that a minimum of a trough has been traversed. Further examination of the data points within each transition region then may be performed to more precisely determine the position (time and amplitude) of each maximum and minimum of the oscillation pattern.

The size of sliding window 122 can be optimized for particular data sets in accordance with the noise level. Since the sliding window spans a range of data points, the window acts as a natural "damper" that reduces the contribution of random noise. Moreover, the slopes do not affect the integrity of the associated data points, and thus do not alter amplitude values or the detection and measurement of valid secondary peaks. Accordingly, use of slopes calculated from subsets of data points selected with a sliding window enables accurate detection and measurement of various parameters of the data without the data corruption that occurs with conventional filtering.

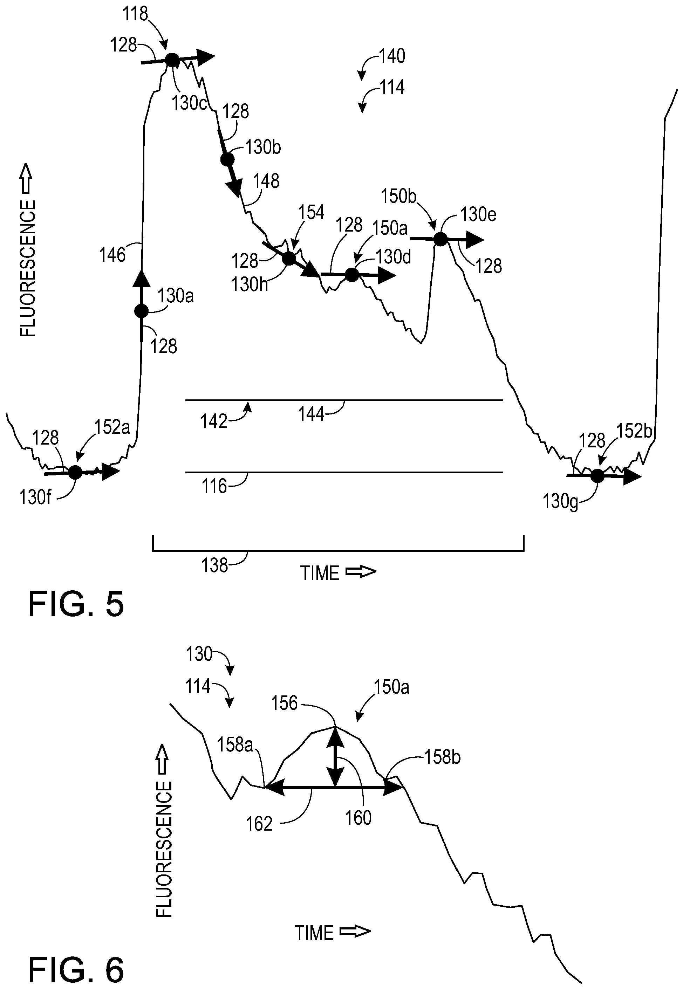

Figure 5 shows a graph of a single oscillation 114 (an "event" 138) of a more complex oscillation pattern 140 detected as in Figure 3. Baseline 116 is positioned at the bottom of oscillation pattern 140. This figure illustrates how vectors 128 having slopes calculated as in Figure 4 can be used to search for larger peaks and troughs, while ignoring smaller "spurious" fluctuations in the fluorescence signal that may result from noise instead of biologically relevant activity. (Vectors 128 are shown here with a constant overall length, not a constant time-component, to simplify the illustration.)

One or more predefined criteria may be used to identify valid events 138 within oscillation pattern 140. Each of the criteria is satisfied by a valid event. The criteria may include at least one amplitude threshold 142 and/or at least one duration threshold to distinguish valid events from other excursions of the fluorescence signal. For example, amplitude threshold 142 may a single point of a trigger level 144 (a line) that is crossed by the fluorescence signal, where an event is characterized by the fluorescence signal crossing the trigger level in both directions. Trigger level 144 may be set at a percentage of the full amplitude span of the oscillation pattern, such as 10%, 15%, 20% of the span from baseline 116. The trigger level may be set automatically and/or by a user, and may be constant or may vary along the time axis (e.g., along a trigger line defining the trigger level). In some cases, a second amplitude threshold may be set to reject each event and/or each peak therein having a maximum with less than a predefined amplitude offset from the baseline (or trigger level 144). The second amplitude threshold may be set at a percentage of the full amplitude span (e.g., 25%, 50%, 60%, 70%, 80%, etc.) or as an absolute value in relative fluorescence units, among others.

During a search along oscillation pattern 140, the beginning of an event 138 is detected when the fluorescence signal crosses trigger level 144 in a direction away from baseline 116. This crossing initiates peak detection. Event 138 is deemed to end when trigger level 144 is crossed in a direction toward baseline 116. However, the left and right troughs around event 138 may be marked below trigger level 144 as explained below.

The slope of vector 128 at each point 130 may be evaluated during the search to determine the direction of transitions. (Exemplary points 130a-130h are shown here.) A positive slope at point 130a is associated with a rising phase 146 of the fluorescence signal and a negative slope at point 130b is associated with a decay phase 148 of the fluorescence signal. The vicinity of the maximum for each peak is detected when the slope of successive vectors 128 changes from positive to negative (with baseline 116 at the bottom). For example, points 130c, 130d, and 130e of vectors 128 are at least very close to the respective maxima for a primary peak 118 and secondary peaks 150a, 150b of the same event 138. The vicinity of the minimum for each trough is detected when the slope of successive vectors 128 changes from negative to positive (with baseline 116 at the bottom). For example, points 130f, 130g of vectors 128 are at least very close to the respective minima for a left trough 152a and a right trough 152b that bound event 138. (Troughs between peaks 118, 150a, and 150b are not expressly identified here to simplify the illustration.)

The size of the sliding window used to create vectors 128 determines the magnitude of low amplitude transitions that will be bypassed by the search. For example, transitions 154 around point 130h are not identified as peaks or troughs in the search because the slopes in this region do not undergo a change in sign (i.e., they remain negative). The greater the size of the window (and thus the length of vectors 128), the larger the transition amplitudes that will be bypassed. Accordingly, vectors 128 collectively act as a filter that does not corrupt the fluorescence signal.

Noise analysis within a narrow region around each transition point can be applied optionally to further improve the detection accuracy of each peak/trough position by rejecting very brief events or "low amplitude" events that might be proximal to a better peak position a few points away.

Figure 6 shows a graph of only a portion of single oscillation 114 (and event 138) of Figure 5 taken around secondary peak 150a. Peaks may be filtered for validity according to one or more criteria, which may be related to a peak maximum 156 and at least one adjacent left or right trough minimum 158a, 158b. For example, the predefined criteria may relate to exceeding a threshold for a local amplitude 160 of each peak (measured between maximum 156 and left minimum 158a and/or right minimum 158b) and/or exceeding a threshold for a duration 162 of the peak, measured at a predefined amplitude (e.g., at left minimum 158a). The thresholds for local amplitude 160 and/or duration 162 may be user-specified or generated automatically from the noise level of the oscillation pattern and/or the sampling interval (i.e., the inverse of the sampling rate) of the oscillation pattern.

### Example 2. Baseline Placement and Noise Estimation

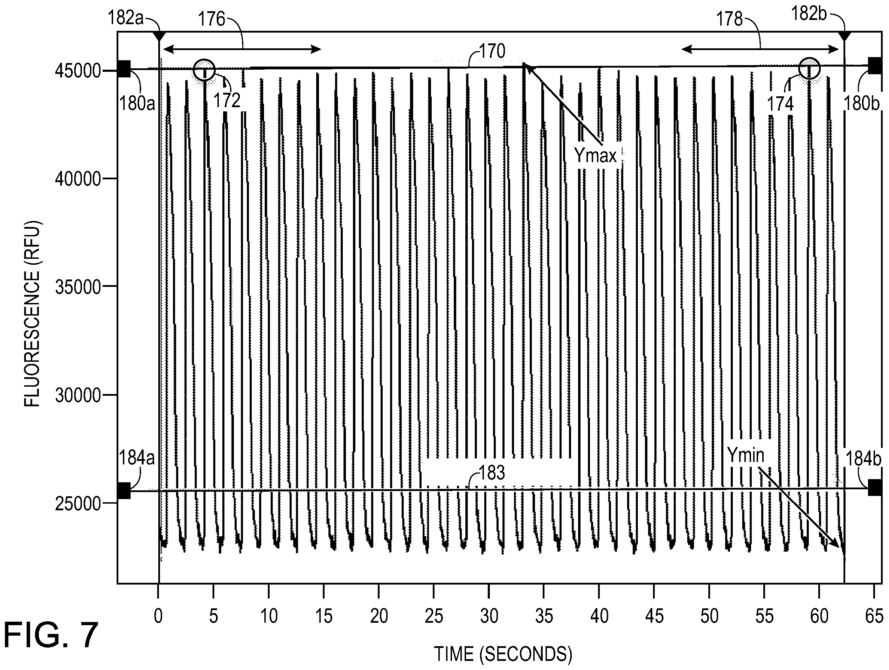

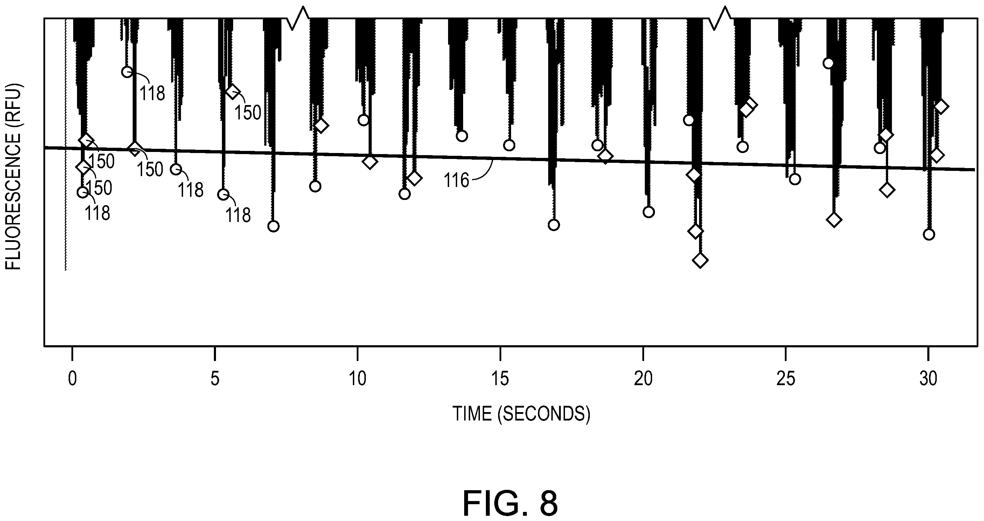

This example describes exemplary approaches to automatically setting a baseline for an oscillation pattern, and to estimating a noise level of the oscillation pattern from negative peaks identified while setting the baseline; see Figures 7 and 8.

The algorithm disclosed herein can estimate a baseline automatically for each data set by measuring the amplitudes of fluctuations that fall within 10% of the data amplitude span, removing outliers by means of interquartile range analysis, and finally fitting a line to the resultant amplitudes with linear regression. The resultant regression line may be suggested or used as the baseline.

Figures 7 and 8 illustrate an exemplary approach to establishing a baseline 116 for a data set. A temporary reference line 170 (e.g., a top baseline) may be set. For example, reference line 170 may be defined by a pair of points 172, 174 respectively representing a maximum amplitude for a leading section 176 and a trailing section 178 of the data set. Each section 176, 178 may represent any suitable fraction of the data set, such as 10, 20, 30, 40, or 50%, among others. Points near the leading end and the trailing end of the oscillation pattern may be excluded, as the ends often include artifacts. The position of reference line 170 may be changed at either or both ends by allowing a user to move handles 180a, 180b via a graphical user interface. Also or alternatively, the user may adjust the time range of the data set deemed to be valid for analysis at either or both ends by moving respective handles 182a, 182b along the time axis via the graphical user interface.

An amplitude span of the data set may be computed. A global amplitude maximum (Ymax) and a global amplitude minimum (Ymin) may be found with a search. The amplitude span is defined as follows: Ymax - Ymin.

A threshold line 183 may be set toward the bottom of the amplitude span opposite reference line 170, such as at 70, 80, 90 or 95% of the amplitude span from reference line 170. For example, a position for threshold line 183 at 90% of the amplitude span from reference line 170 may be calculated as follows: Ymin + 0.1 x (amplitude span). The user may adjust threshold line 183 at either or both ends by moving respective handles 184a, 184b along the amplitude axis via the graphical user interface.

A search for peaks with respect to reference line 170 may be performed (see Figures 7 and 8). In the present example, the search finds negative peaks by identifying local maximum excursions away from (below) reference line 170 that also are located below threshold line 183. (In other words, this peak search is the inverse of that described above for Figure 5.) The search may be performed using a series of slopes as described above for Figures 4 and 5, with the slopes obtained using a relatively small size for the sliding window (e.g., a width of 3, 4, or 5 data points). A small sliding window provides a high sensitivity for peak detection, with minimal elimination of noise. Accordingly, the noise level for the data set also may be estimated from amplitude values of the peaks found in the search.

Primary peaks 118 and secondary peaks 150 may be identified. Here and in examples to follow, circles mark the maxima of primary peaks 118, and diamonds mark the maxima of secondary peaks 150 (see Figure 8). These two types of peaks 118, 150 also may be marked distinguishably when the oscillation pattern is graphed and displayed to the user (e.g., via a graphical user interface). However, for setting the baseline, all detected peaks may be recorded and treated equivalently, that is, without any distinction between primary and secondary peaks.

Using linear regression, a straight line may be fitted through the detected peak amplitudes. This line may be taken as the initial approximation of baseline 116. The trigger level 144 initially may be set at 10% of the data span above the baseline. These automatically assigned levels can subsequently be adjusted by the user via a graphical interface or by explicitly setting values.

Prior to linear regression, the data points at the maxima of peaks 118, 150 may or may not be filtered. For example, before choosing a baseline, outliers may be removed, such as using interquartile range analysis. Points more than 1.5 interquartile ranges below the first quartile or above the third quartile may be rejected..

A noise level may be estimated from peaks 118, 150 (see Figure 8). For example, a noise estimate may be calculated as the mean of successive differences between the amplitudes of peaks 118, 150 (considered as a group). The noise estimate may be used directly as the noise level or may be used to calculate a noise level. This noise level, alone or in conjunction with the sampling interval, may enable automatic selection of a suitable size for the sliding window (see Example 2) for a given data set. An exemplary algorithm for this size selection is heuristic: [0.3 / sampling interval (in seconds)], added to a "noise factor" of [noise level / 100]. The algorithm may select a window size from a range of permitted sizes. The range may include only odd numbers of data points (e.g., 3, 5, 7, etc.), only even numbers of data points (e.g., 4, 6, 8, etc.), or odd and even numbers (e.g., 3, 4, 5, 6, etc.). The noise level also may be utilized to compute an automatically generated value for a suitable threshold for local amplitude 160, to reject smaller peaks as invalid (also see Examples 1 and 5). The data sampling rate may be used to automatically generate a suitable threshold for duration 162, to reject very short-lived peaks as invalid.

### Example 3. Parameters of Interest

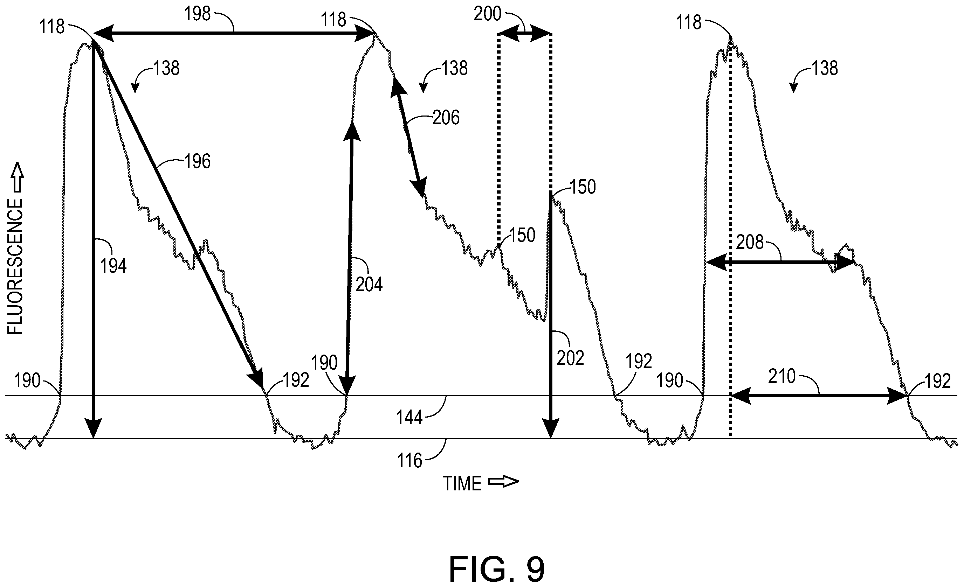

This example describes exemplary parameters of interest that can be measured from an oscillation pattern using the algorithms described herein; see Figure 9.

Three events 138 of the oscillation pattern are shown. A starting point 190 and an ending point 192 are marked for each event 138, where the fluorescence signal crosses trigger level 144 in opposite directions.

Any of the parameters described herein may be reported for individual peaks/events or may be averaged over a series of peaks/events of a given oscillation pattern. Each primary peak 118 of an event 138 has a primary peak amplitude 194. The primary peak amplitude can be measured as the amplitude difference between the maximum of primary peak 118 and baseline 116. A linear decay slope 196 may be defined by a straight line extending between the maximum of primary peak 118 and ending point 192 for the event 138.

A primary peak interval 198 (also called a primary peak period) is defined between maxima of adjacent primary peaks 118. A series of events 138 define a succession of primary peak intervals 198, which may be converted to a primary peak rate for the series of events, expressed as primary peaks per unit time, such as peaks per minute (PpM).

A secondary peak period 200 is defined between maxima of adjacent secondary peaks 150 within or between events. The secondary peak period 200 for the oscillation pattern may be converted to a secondary peak rate, expressed as secondary peaks per unit time, such as peaks per minute (PpM). Each secondary peak 150 has a secondary peak amplitude 202, which may be measured between the maximum of the secondary peak and baseline 116.

A rise slope 204 and a decay slope 206 may be calculated for each event 138. Each of these slopes may be defined between percentages of the maximum value of the event, such as between 10-80% or 30-70% of the maximum value.

A duration 208 of each event 138 may be calculated at a specified percentage from the maximum amplitude of the event, such as at 10, 20, 30, 40, 50, 60, 70, 80, or 90%, among others, from the maximum amplitude. Alternatively, or in addition, a duration from peak 210 may be measured for each event starting at the maximum of the primary peak 118 of the event. The duration from peak may be measured at a specified percentage from the maximum value of the event, such as 10, 20, 30, 40, 50, 60, 70, 80, or 90%, among others, from the maximum value.

### Example 4. Effect of Window Size on Peak Detection

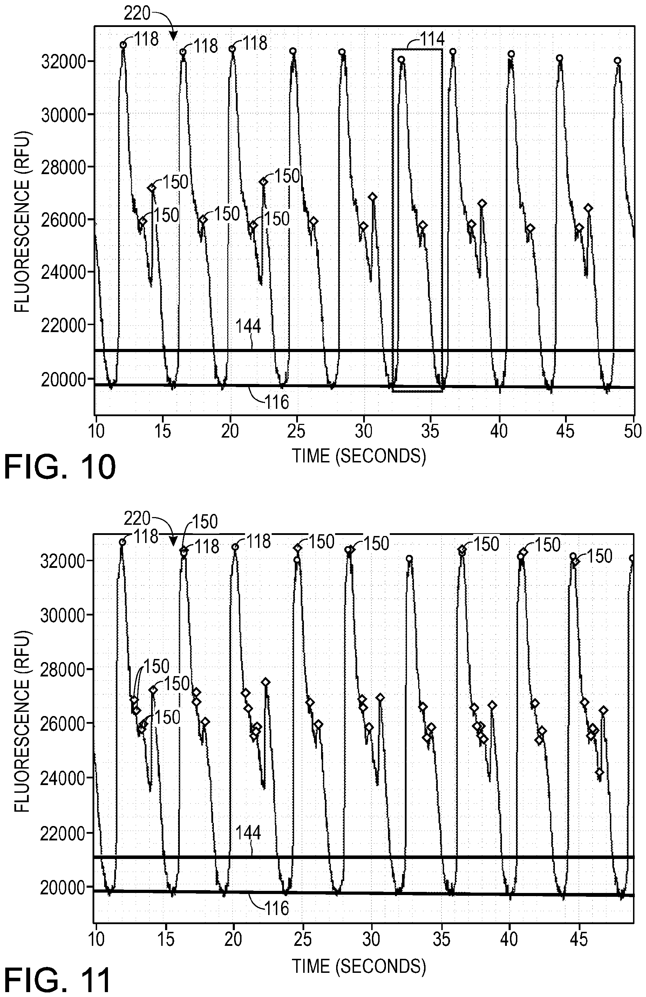

This example describes the effect of changing the window size on the sensitivity of peak detection; see Figures 10 and 11.

The performance of a peak-finding algorithm based on slopes calculated as in Example 1 was tested on fluorescence intensity data collected from beating cardiomyocytes. The cardiomyocytes were labeled in vitro with a florescent calcium indicator and treated with a cardiotoxic compound to perturb the regular calcium oscillations present in control cardiomyocytes (e.g., see Example 1). The same data are graphed in Figures 10 and 11 and produce a complex oscillation pattern 220. The pattern is composed of a series of oscillations 114 each exhibiting a more prolonged, erratic decay phase relative to the oscillations of control cells. The decay phase is characterized by early afterdepolarization (EAD)-like peaks (i.e., secondary peaks).

A peak search was performed on the data with an automatically-determined sliding window size of eleven points (Figure 10), or a user-selected window size of three points (Figure 11). A primary peak 118 of each oscillation is marked with a circle, and secondary peaks 150 are marked with diamonds. In Figure 10, with a larger window size, low amplitude transitions are ignored. In Figure 11, with a smaller window size, low amplitude transitions are detected as peaks. Accordingly, the sensitivity of peak detection can be controlled by judicious selection of the window size, optionally in conjunction with noise rejection to deem detected peaks that are too small/transient as invalid (e.g., see Examples 1 and 5).

### Example 5. Dialog Box for Analysis Software

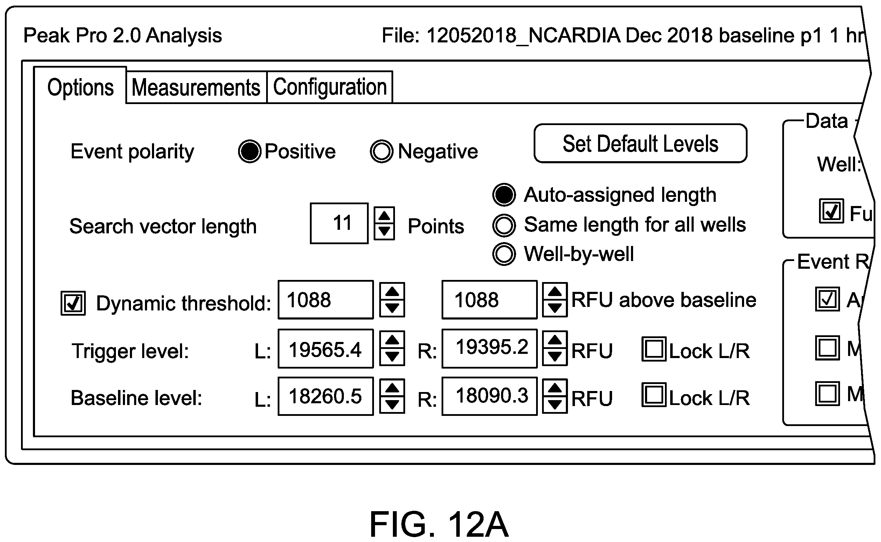

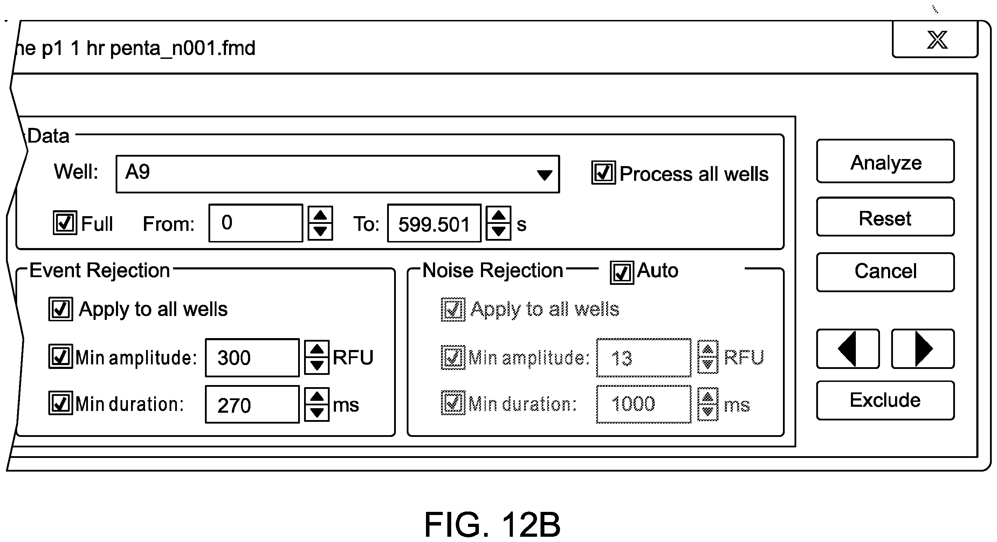

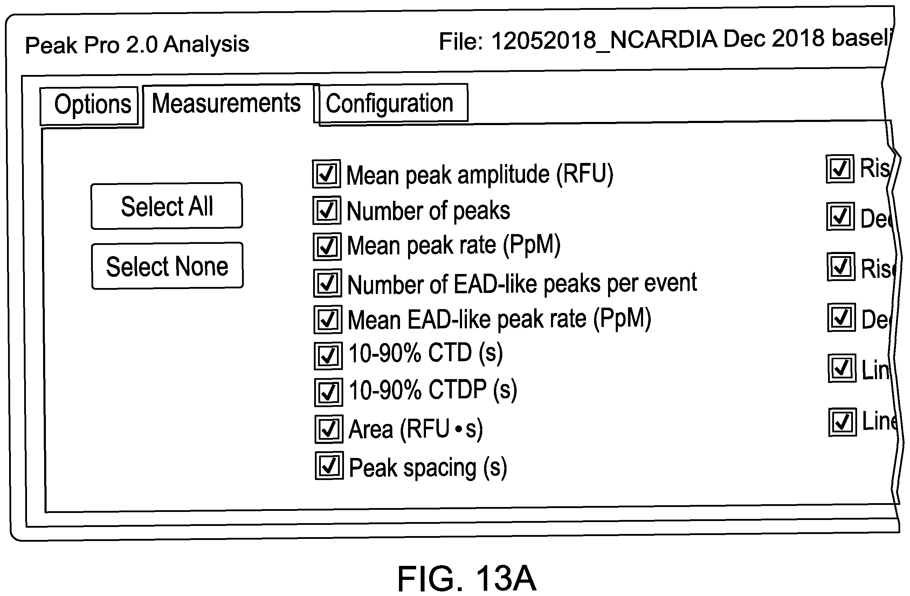

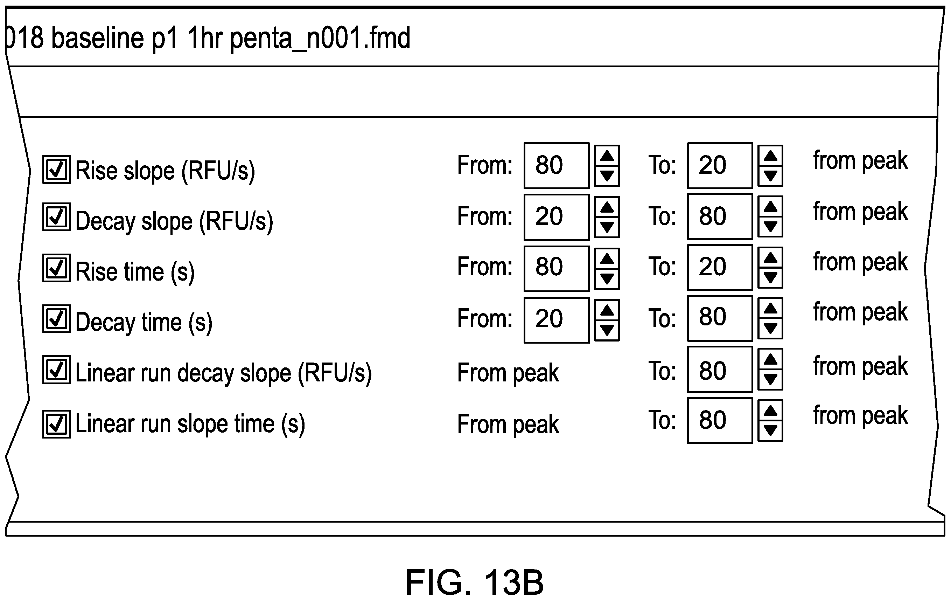

This example describes an exemplary dialog box displayed by a graphical user interface to facilitate user input to the analysis software; see Figures 12A, 12B, 13A, and 13B (also see Figure 9).

Figures 12A, 12B, 13A, and 13B show screenshots of the dialog box, which controls analysis optimization and the list of readouts (i.e., descriptors) of peak analysis. The dialog box contains two tabs. The "Options" tab provides access to the analysis settings and the data range. The "Measurements" tab contains the properties of an event that can be selected for display in an output sheet. Exemplary aspects and features of these tabs are described below.

### Options tab:

Event polarity. Two buttons, Positive and Negative, are available to choose the event polarity (see Figure 12A). Positive events involve an increase in fluorescence above a bottom baseline, and negative events involve a decrease in fluorescence below a top baseline.

Select vector length. The search vector length (i.e., the size of the sliding window) may be the most important variable for good peak detection (see Figure 12A; also see Figures 4 and 5). Search vectors are generated from a series of subsets of the data points of a data set, where the subsets are selected by incrementally moving the sliding window along the time axis. A search vector is generated at each point. Vectors are computed using both forward-looking and backward-looking data points, centered about the window width and ranging from -((Window Width) -1)/2 to +((Window Width) -1)/2. Slopes near the opposite ends of the data set are computed using appropriately declining numbers of data points. A linear regression is performed for each subset of data points to generate the series of search vectors. In other words, the sliding window slides along the data set, to check the slope at each data point, and determines the presence of transitions (maxima/peaks and minima/troughs) by noting a change in direction of the slope (i.e., a sign change of the slope from positive to negative or negative to positive). The length of the search vector relative to the sampling rate determines the sensitivity of detecting transitions. Longer vector lengths will automatically filter out noise (i.e., events/peaks of lower amplitude and/or shorter duration), while shorter vector lengths will detect transitions of lower amplitude and shorter duration.

Three buttons are available for choosing how the vector length is assigned: "Auto-assigned length," "Same length for all wells," and "Well-by-well" (see Figure 12A). The software uses the sampling rate and an estimate of the noise amplitude to automatically compute a vector length when "Auto-assigned length" is chosen. Automatically-generated vector lengths are generally a good starting point for data analysis. The user can select a different vector length for each well (i.e., for each set of data points describing each different oscillation pattern), if the "Well-by-well" button is chosen.

Dynamic threshold. This option sets a lower limit for valid peaks (see Figure 12A). All detected peaks having a peak amplitude below this threshold will be deemed invalid and ignored. The value of this limit is given as relative fluorescence units (RFU) above baseline in the present example. Two boxes are available for entering different threshold amplitude values above baseline for the left end and the right end of a line defining the dynamic threshold. In other examples, the dynamic threshold may be set as a percentage of the amplitude span of the data set. For example, if the data points for a given well span a range of 10,000 relative fluorescent units (RFU), a setting of 80% will specify a cutoff limit of 8,000 RFU.

Trigger level. This level is defined by a trigger line extending along the time axis and determines when an event is deemed to start and finish (see Figure 12A). An "event" is defined by the fluorescence signal crossing the trigger line in both directions. The amplitude of the event, however, is measured relative to the baseline. The detection of the left and right troughs of the event is not limited by the trigger level; these measurements can extend as far as the baseline. The event duration can be measured as the temporal distance between the two flanking trough positions, or by the temporal distance between the two crossing points for the event on the trigger line, among others. The trigger level is set by default at 10% above the baseline (relative to the data range). This level can be set and changed individually for each dataset (well data).

Baseline level. The baseline level defines the lower end of the detection range and the position of the baseline. All amplitudes are measured relative to the baseline level and not a trough value.

The level names (Dynamic threshold, Trigger level, and Baseline level) are color-coded in the dialog box to match the color of corresponding lines in the graph window. Each of these levels can be defined by a respective line having a left end and a right end. The ends can be moved separately by grabbing handles at the ends with the left mouse button and moving up or down. The entire level line can be moved up or down by grabbing the line anywhere away from the handles with the left mouse button and moving up or down. The level line positions also can be set by changing the values in the respective L (left) or R (right) edit boxes, either by means of the spinner or entering a value directly. The "Lock" feature will cause the line ends to move in unison upon adjustment of either the right or left edge values. This feature applies only to the dialog box and does not affect the positioning by mouse.

Set Default Levels. Pressing this button will reset the baseline and trigger levels to their default positions (see Figure 12A).

Process all wells (see Figure 12B). If this item is checked, then all selected well data will be processed. If unchecked, only the currently selected well will be processed.

The Data Group (see Figure 12B):
Well. Analysis will be applied only to the wells that are selected and that appear in the "Detail" graph window. Only the selected well appears in the graph at any given time. The well data to display can be specified by selecting the respective data series in the "Well" combo box.

The Event Rejection Group (see Figure 12B):
Min amplitude. This item specifies the minimum amplitude, as measured from the baseline, that will be accepted as valid. Excursions forming primary/secondary peaks having maxima below the minimum amplitude value will be rejected. The minimum amplitude is substantially the same as the "Dynamic threshold" (see Figure 12A), except that the minimum amplitude is a single value instead of being defined by values along a line having left and right ends that are independently adjustable.

Min duration. This item specifies the minimum duration of an event that will be accepted as valid (see Figure 12B). The event duration may be measured between the left and right trough values, or between the beginning and the end of the event, as defined by crossing the trigger level. Excursions with durations shorter than the minimum duration value will be rejected.

Apply to all wells. If this item is checked, the amplitude and duration values that appear in the edit boxes will apply to the data from all wells. If unchecked, the value specified for each individual well will be used.

The Noise Rejection Group (see Figure 12B):
Min amplitude. This item specifies a minimum amplitude threshold for the local amplitude of valid peaks. The local amplitude for a peak (valid or invalid) is measured as the amplitude difference between the amplitude value of the peak and the amplitude value of the nearest left (and/or right) trough. Peaks having a local amplitude below the minimum amplitude threshold will be rejected as invalid.
Min duration. This item specifies a minimum duration threshold for valid peaks. The duration for a peak (valid or invalid) is measured as the absolute value of the time difference between (a) the time value for the lower of the two adjacent troughs and (b) the time value for the equivalent amplitude on the opposite side of the peak. Peaks having a duration shorter than minimum duration threshold will be rejected as invalid.
Apply to all wells. If this item is checked, the values for the amplitude and duration thresholds that appear in the edit boxes will apply to the data from all wells (i.e., all of the data sets for separate oscillation patterns). If unchecked, the values specified for each individual well will be used.
Auto. The software measures the approximate noise level of each data set. First, the peaking-finding algorithm, using a short search vector length (such as 3), locates all peaks ("noise peaks") within a percentage range (e.g., 0-10%) of the amplitude span of the data set from the baseline. The noise peaks may have maxima relative to a reference line opposite the baseline. (For example, with a bottom baseline, the noise peaks may be negative peaks having maxima relative to a reference line located at the top of the oscillation pattern (see Example 2).) A noise estimate is then computed as the mean of the absolute difference between successive maximum values of the noise peaks falling within an interquartile range. The noise level is taken as 30% of the noise estimate. The noise estimate and noise level are only reasonable if the data are relatively clean and most of the noise is located near the baseline.

### Measurements Tab (see Figures 13A and 13B):

This tab is used to specify those measurements that are to be reported in the statistics page. This page displays the results for each well (group), the mean of events that are detected in the well, and the individual values for each event, where applicable.

Select All. This button selects all of the events for display (see Figure 13A).

Select None. This button clears the event display selection.

Mean peak amplitude. The mean peak amplitude is the average amplitude of the maxima of valid primary peaks of the events detected for a given well, expressed in relative fluorescence units (RFU). Each peak amplitude for calculating the average is measured between the maximum of the peak and the baseline.

Number of peaks. This descriptor is the total number of valid primary peaks or the total number of valid primary and valid secondary peaks combined for a given well.

Mean peak rate. This descriptor is the number of valid primary peaks per minute (PpM). For cardiac data this is equivalent to "beats per minute" (BPM).

Number of EAD-like peaks per event. This descriptor is the number of valid secondary peaks (EAD-like peaks) in an event or for the data set. This item reports the mean number of valid secondary peaks per event, the standard deviation of the mean, and the total number of valid secondary peaks for the data set.

Mean EAD-like peak rate (PpM). This descriptor is the average rate of the valid secondary peaks (EAD-like peaks) detected in the data set (for a given well) expressed in peaks per minute (PpM).

Number of EAD-like peaks S.D. The standard deviation of the number of EAD-like peaks per event.

10-90% CTD. These descriptors are the duration of an event (e.g., a Calcium Transient Duration) in seconds at the specified level from the maximum of the primary peak of the event to the baseline. For example, CTD10 is the duration of the event measured at an amplitude level that is 10% of the amplitude distance from the maximum to the baseline. CTDs from 10% at the top to 90% at the bottom are measured. At times, troughs are at some distance from the baseline, obviating the measurement of CTDs farthest from the peak.

10-90% CTDP. These descriptors are the duration of an event from the temporal position of the maximum of the primary peak to the specified level from the maximum relative to the baseline. (CTDP is Calcium Transient Duration from Peak.) For example, CTDP10 is the duration of the event from the peak at a level of 10% of the amplitude distance from the maximum of the peak to the baseline. CTDs from 10% to 90% are measured. At times, troughs are at some distance from the baseline, obviating the measurement of CTDPs farthest from the peak.

Area. The area between the oscillation trace and the baseline for a given event, measured from the start to the end of the event, as defined by the left and right troughs for the event. The units for the area are RFU•seconds.

Peak Spacing. This measurement determines the regularity of the spacing of valid primary peaks (in seconds) by comparing the standard deviation and the mean of the spacing. The measurement reports either uniform spacing of peaks (OK), if the standard deviation is less than a threshold percentage (e.g., 50%) of the mean, or irregular spacing (IRREG), if the standard deviation is greater than the threshold percentage.

Slopes. The percentages to and from the peaks for slope measurement are specified in the associated edit boxes. Typically, slopes are measured between 10-80% or 30-70% from the peak values.

### Example 6. Compound Testing with Cardiomyocytes

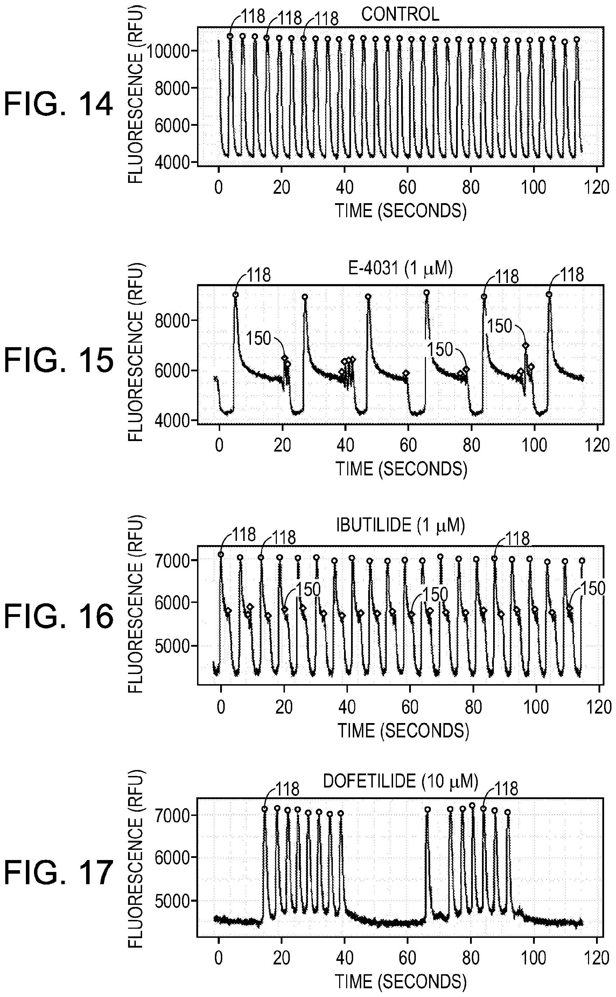

This example describes results obtained from testing cardiotoxic compounds on cardiomyocytes for effects on various parameters measured by the software described in Example 5; see Figures 14-22.

Development of biologically relevant and predictive cell-based assays for compound screening and toxicity assessment is a major challenge in drug discovery. The focus of this study was to establish high-throughput-compatible cardiotoxicity assays using human-induced pluripotent stem cell (iPSC)-derived cardiomyocytes. To assess the utility of human iPSC-derived cardiomyocytes as an in vitro pro-arrhythmia model, the concentration dependence and responses were evaluated for 28 drugs linked to low, intermediate, and high torsades de pointes (TdP) risk categories. (The compounds were suggested by the Comprehensive in Vitro Proarrhythmia Assay (CiPA) Initiative.) The impact of various compounds on the contraction rates and patterns of cardiomyocyte spontaneous activity was monitored by changes in intracellular Ca²⁺ oscillations measured by fast kinetic fluorescence with calcium-sensitive dyes. Advanced image analysis methods were implemented to provide multi-parametric characterization of the Ca²⁺ oscillation patterns. This assay allows for the characterization of parameters such as beating frequency, amplitude, peak width, and rise and decay times. The results demonstrate the utility of hiPSC cardiomyocytes for detecting drug-induced proarrhythmic effects in vitro.

The iPSC-derived cardiomyocytes generate spontaneous synchronized calcium oscillations. High speed fluorescence imaging with the FLIPR^{®} Penta^{®} System was used to measure the patterns and frequencies of the Ca²⁺ oscillations in cardiomyocytes as monitored by changes in intracellular Ca²⁺ levels with the EarlyTox^{™} Cardiotoxicity Kit. A set of 28 known cardiotoxic compounds, plus several benchmark compounds and negative controls, were tested in the assay.

iPSC-derived Cardiomyocytes: Cryopreserved human iPSC-derived cells from Cellular Dynamics International (CDI) iCell^{®} Cardiomyocytes2 were used for experiments. Cells were thawed and plated at 20,000/well (96-well format) or 10,000/well into 384-well format plates (Corning) and incubated for 7 days in maintenance media. The presence of strong synchronous contractions in the 3D cultures was confirmed visually prior to running experiments. In addition, assay-ready 384-Well plates with cardiomyocytes1 were obtained from Ncardia, Inc. Plates were shipped pre-plated and allowed to recover after arrival for 2 days.

Cardiomyocytes were exposed to compounds for 15, 30, 60, or 90 min, or 24 hours.
The intracellular Ca²⁺ oscillations were assessed using the EarlyTox^{®} Calcium dye (Molecular Devices) according to the regular protocol; cells were loaded with dye for 2 hours before measurements.

Measurement of calcium oscillation in iPSC-derived cardiomyocytes is a promising method for toxicity assessment. The present work focuses on evaluation of a set of 28 CiPA compounds categorized as High, Medium, or Low risk according to clinical data.

The FLIPR^{®} Penta^{®} System equipped with a new high speed camera allows better resolution of calcium oscillation patterns in cardiomyocytes. The ScreenWorks^{®} Peak Pro^{™} 2 software allows complex event analysis and detailed characterization of patterns using more than 20 available pattern descriptors. The assay can be used for testing developing drugs and screening chemicals for potential cardiotoxic hazards.

Figures 14-21 show representative traces of calcium oscillations for control and compound-treated cardiomyocytes recorded for two minutes, starting after treatment for thirty minutes with the indicated compounds and concentrations. Perturbations to the calcium oscillation pattern are described for each of the compounds.

A control trace (DMSO treatment of cardiomyocytes) is shown in Figure 14. Only primary peaks 118 (marked with circles) are detectable. The primary peaks have a uniform amplitude and spacing from one another.

Figure 15 shows a trace from cardiomyocytes treated with E-4031 at a concentration of 1 µM. Primary peaks 118 and secondary peaks 150 are present. The primary peaks are prolongated.

Figure 16 shows a trace from cardiomyocytes treated with ibutilide at a concentration of 1 µM. Primary peaks 118 and secondary peaks 150 are present. The primary peaks are prolongated.

Figure 17 shows a trace from cardiomyocytes treated with dofetilide at a concentration of 10 µM. Only primary peaks 118 are detected, but with an irregular spacing from one another.

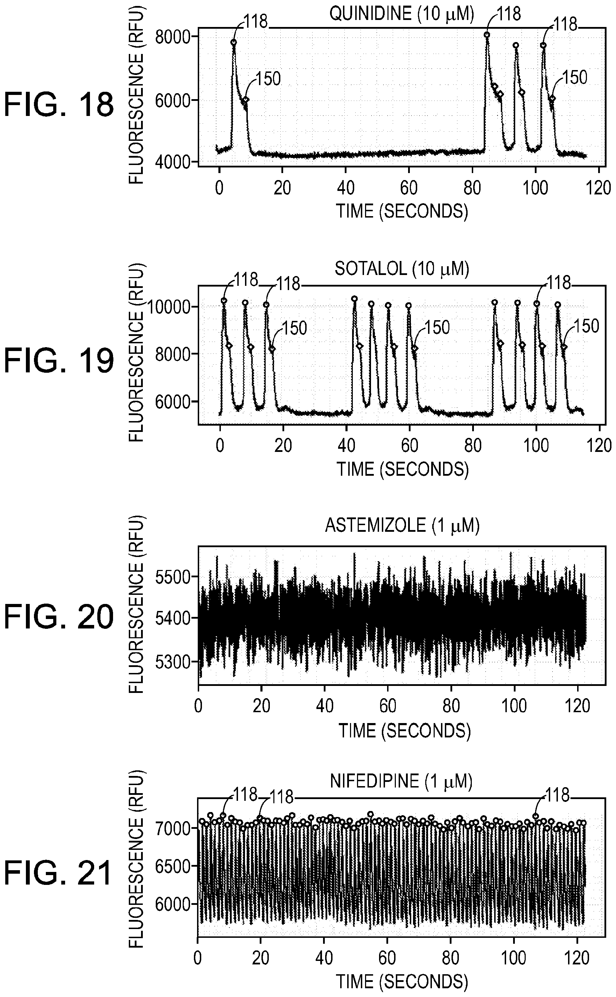

Figure 18 shows a trace from cardiomyocytes treated with quinidine at a concentration of 10 µM. Primary peaks 118 and secondary peaks 150 are detected. The primary peaks are prolongated and have any irregular spacing from one another.

Figure 19 shows a trace from cardiomyocytes treated with sotalol at a concentration of 10 µM. Primary peaks 118 and secondary peaks 150 are detected. The primary peaks are prolongated and have any irregular spacing from one another.

Figure 20 shows a trace from cardiomyocytes treated with astemizole at a concentration of 1 µM. No valid peaks are detected and thus a "stop" oscillations condition is reported.

Figure 21 shows a trace from cardiomyocytes treated with nifedipine at a concentration of 1 µM. Only primary peaks 118 are detected, but at a significantly higher frequency than control.

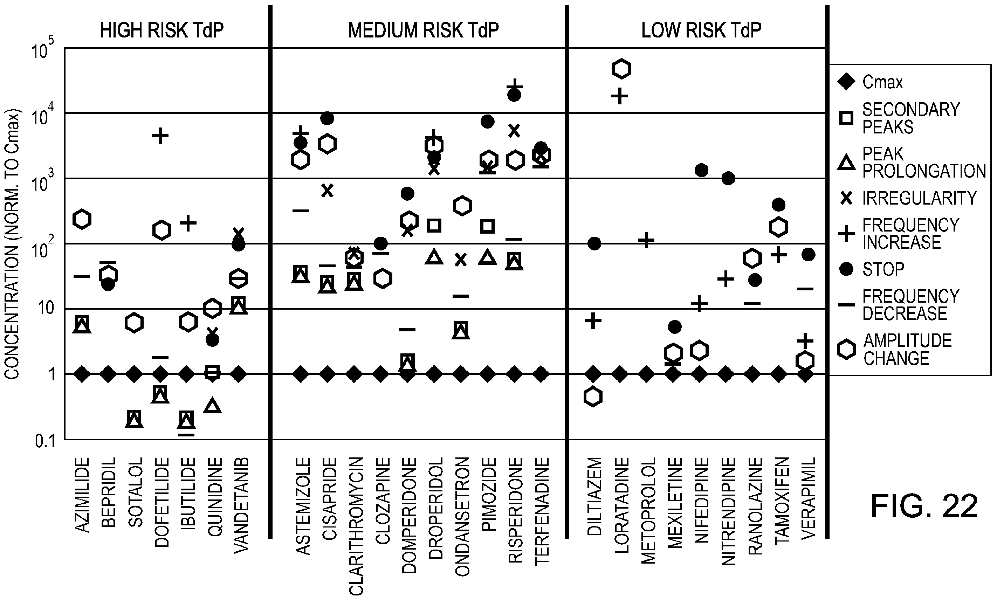

Figure 22 shows a compilation and comparison of different readouts for the compounds over a range of normalized concentrations, with compounds grouped according to known cardiotoxicity into three groups: high toxicity, medium toxicity, and low toxicity. The readouts include the presence of secondary peaks, peak prolongation, irregularity in the spacing/amplitude of primary peaks, a frequency increase in primary peaks relative to control, a stop condition (no valid peaks), a frequency decrease in primary peaks relative to control, and an amplitude change of primary peaks within the oscillation pattern. The graph demonstrates that the appearance of secondary peaks and peak prolongations at concentrations comparable to Cmax (the maximum clinical concentration in blood) is apparently a strong indicator of (or correlates with) cardiac toxicity, while modifications of other readouts do not necessarily indicate cardiotoxic effects.

### Example 7. Compound Testing with Neurons

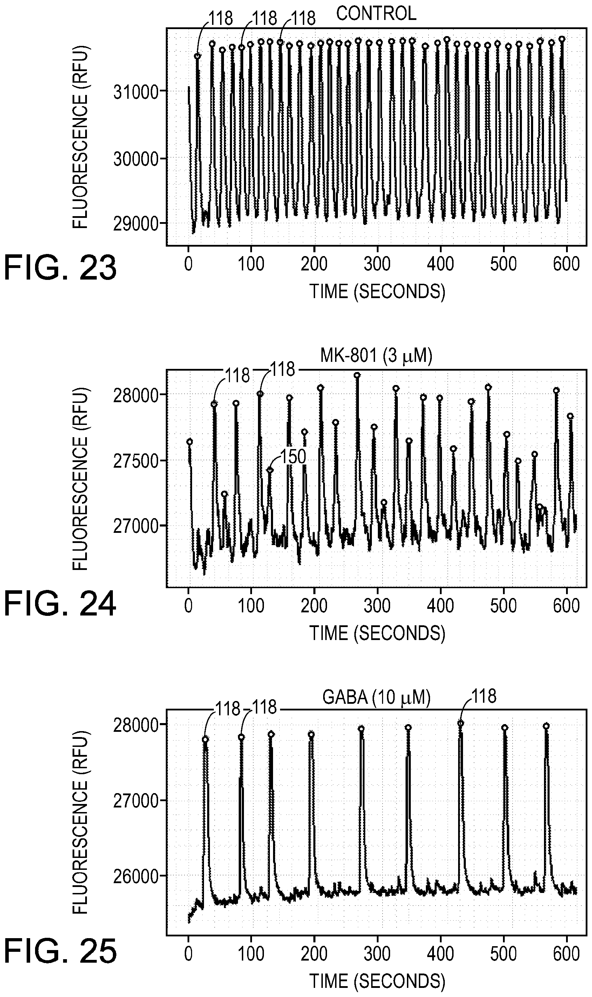

This example describes results obtained from testing neurotoxic compounds with neurons in vitro for effects on various parameters measured by analysis software, see Figures 23-30.

To speed up the development of more effective and safer drugs, there is an increasing need for more complex, biologically relevant, and predictive cell-based assays for drug discovery and toxicology screening. Human iPSC-derived neural 3D co-cultures (StemoniX^{®} microBrain^{®} 3D platform) have been developed as a high-throughput screening platform that more closely resembles the constitution of native human cortical brain tissue. Neural spheroid 3D co-cultures are a physiologically relevant co-culture of iPSC-derived, functionally-active cortical glutamatergic and GABAergic neurons co-differentiated and matured with astrocytes from the same donor. 3D neural spheroids contain a neural network enriched in synapses, creating a highly functional neuronal circuitry and display spontaneous synchronized, readily detectable calcium oscillations.

A new method is disclosed herein for the analysis of complex calcium oscillations. The method allows detection and multi-parametric characterization of oscillation peaks. The multi-parametric characterization may include the oscillation rate (i.e., the frequency of primary peaks), width and amplitude of primary peaks, descriptors of secondary peaks, waveform irregularities, and several other important readouts.

The neuronal cells in the microBrain^{®} 3D spheroids generate spontaneous synchronized calcium oscillations. Fast kinetic fluorescence imaging by the FLIPR^{®} Penta^{®} system was employed to measure the patterns and frequencies of Ca²⁺ oscillations of neuro-spheroids, as monitored by changes in intracellular Ca²⁺ levels with the FLIPR^{®} Calcium 6 Assay Kit. A set of known neuromodulators was tested, including agonists and antagonists of NMDA, GABA and AMPA receptors; kainic acid; and analgesic and anti-epileptic drugs. The fluorescence from each well of an entire 384-well plate was recorded simultaneously at a frequency of 2 Hz (0.5 second sampling interval) by high-speed imaging using the FLIPR^{®} Penta^{®} system.

Advanced analysis methods implemented in the ScreenWorks^{®} Peak Pro^{™} 2 software module provide multi-parametric characterization of Ca²⁺ flux oscillation patterns. This phenotypic assay creates readouts such as oscillation frequency, amplitude, peak width, peak rise and decay times, and peak amplitude/spacing irregularity. The effects of modulators of neuronal activity were evaluated by measuring changes in several measurements.

A set of over twenty compounds, including a number of known modulators of neuronal activity, was assayed at different time points and concentrations, and the EC50 values were calculated for compound effects. The time points included 0, 15, 30, 60, 90, and 120 minutes, and 24 hours. Changes were observed as inhibitions or activations of the peak frequency, or other measurements, corresponding to the expected effect of the corresponding neuromodulator.

Figures 23-30 show representative traces of calcium oscillations for control and compound-treated neural spheroids recorded for ten minutes, starting after 30 minutes of treatment with the indicated compounds and concentrations.

A control trace (DMSO treatment of a spheroid) is shown in Figure 23. Only primary peaks 118 (marked with circles) are detectable. The primary peaks have a fairly uniform amplitude and spacing from one another.

Figure 24 shows a trace from a spheroid treated with MK-801 at a concentration of 3 µM. Primary peaks 118 have a variable amplitude (compare with Figure 23). Only one secondary peak 150 is detected.

Figure 25 shows a trace from a spheroid treated with GABA at a concentration of 10 µM. Only primary peaks 118 are detected, but at a significantly lower frequency than control.

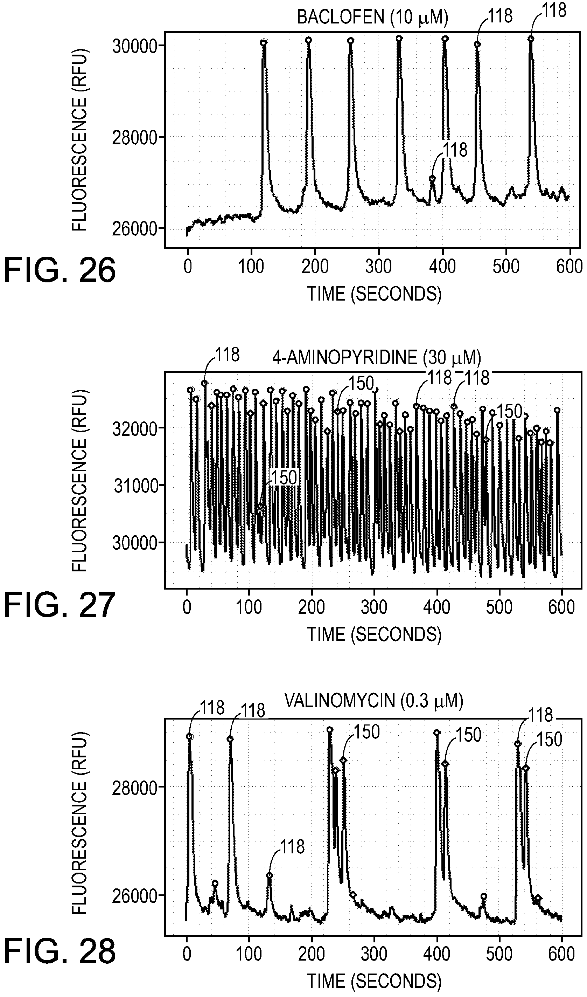

Figure 26 shows a trace from a spheroid treated with baclofen at a concentration of 10 µM. Only primary peaks 118 are detected, but at a significantly lower frequency than control and with an irregular spacing and amplitude.

Figure 27 shows a trace from a spheroid treated with 4-aminopyridine at a concentration of 30 µM. Primary peaks 118 and secondary peaks 150 are detected. The frequency of primary peaks 118 is increased relative to control.

Figure 28 shows a trace from a spheroid treated with valinomycin at a concentration of (0.3 µM). Primary peaks 118 and secondary peaks 150 are detected. The frequency of primary peaks 118 is much less than control, and the amplitude and spacing are much more variable.

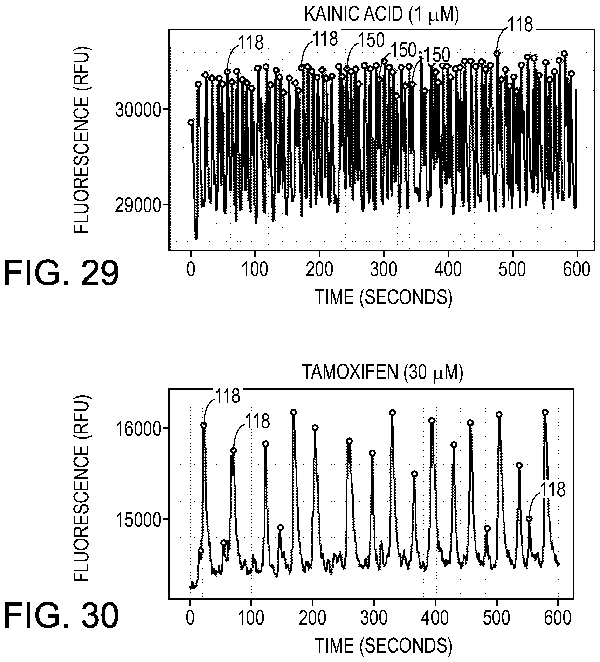

Figure 29 shows a trace from a spheroid treated with kainic acid at a concentration of 1 µM. Primary peaks 118 and numerous secondary peaks 150 are detected. The frequency of primary peaks 118 is increased relative to control.

Figure 30 shows a trace from a spheroid treated with tamoxifen at a concentration of 30 µM. Only primary peaks 118 are detected, but at a significantly lower frequency than control and with a variable amplitude.

The assay can be used for testing compound effects and screening for neurotoxic chemicals. The appearance of secondary peaks and low-amplitude primary peaks can indicate compounds with neurotoxic activity. Effects on peak rate and amplitude alone may not be sufficiently predictive of neurotoxicity.

The term "exemplary" as used in the present disclosure, means "illustrative" or "serving as an example." Similarly, the term "exemplify" means "illustrate by giving an example." Neither term implies desirability nor superiority.

The disclosure set forth above may encompass multiple distinct inventions with independent utility. Although each of these inventions has been disclosed in its preferred form(s), the specific embodiments thereof as disclosed and illustrated herein are not to be considered in a limiting sense, because numerous variations are possible. The invention is only limited by the appended claims.

## Claims

1. A method of analysis, the method comprising:
detecting (54) fluorescence (91) representing an oscillating ion flux associated with one or more biological cells (72), to produce a series of data points (120) describing an oscillation pattern (110), wherein the oscillation pattern (110) comprises a series of events (138), wherein the oscillation pattern (110) crosses a predefined trigger level (144) twice for each event (138), and wherein the trigger level (144) is set relative to a baseline (116) of the oscillation pattern (110);
identifying (62) primary peaks (118) and secondary peaks (150a, 150b) in the oscillation pattern (110), wherein each event (138) includes a single primary peak (118) and wherein each of the secondary peaks (150a, 150b) is included in an event of the series of events (138), and wherein each secondary peak is any peak following the primary peak within an event; and
determining an aspect of the secondary peaks (150a, 150b);
wherein detecting, identifying, and determining are performed for each separate set of a plurality of separate sets of one or more biological cells (72), and wherein each separate set is exposed to a different compound or to a different concentration of the same compound;
further comprising determining an effect, if any, of each different compound or concentration on the aspect of the secondary peaks (150a, 150b); and
predicting a degree of cardiotoxicity or neurotoxicity of each compound or concentration based on the aspect of the secondary peaks, and a spacing or amplitude regularity or irregularity of the primary peaks or a number, frequency, or period of primary peaks that are smaller peaks having an amplitude below a predefined threshold.

2. The method of claim 1, wherein determining an aspect of the secondary peaks (150a, 150b) includes determining a number, frequency, or period of the secondary peaks (150a, 150b).

3. The method of claim 1 or 2,
further comprising determining a spacing regularity/irregularity of the primary peaks (118) and/or, an amplitude regularity/irregularity of the primary peaks (118); or
further comprising comparing an amplitude (194) of each primary peak (118) to a predefined threshold to enumerate smaller peaks, if any, of the primary peaks.

4. The method of claim 1, 2 or 3, further comprising labeling (52) the one or more biological cells (72) with a calcium indicator, wherein the fluorescence (91) is emitted by the calcium indicator.

5. A system (70), comprising:
an optical sensor (88) configured to detect fluorescence (91) representing an oscillating ion flux associated with one or more biological cells (72), to produce a series of data points (120) describing an oscillation pattern (110); and
a processor (96) configured to (1) identify primary peaks (118) and secondary peaks (150a, 150b) in the oscillation pattern (110), and (2) to determine an aspect of the secondary peaks (150a, 150b) for an
oscillation pattern (110) comprising a series of events (138), wherein the oscillation pattern (110) crosses a predefined trigger level (144) twice for each event (138), and wherein the trigger level (144) is set relative to a baseline (116) of the oscillation pattern (110);
wherein each event (138) includes a single primary peak (118) and wherein each of the secondary peaks (150a, 150b) is included in an event of the series of events (138), wherein each secondary peak is any peak following the primary peak within an event; and
wherein detecting, identifying, and determining are performed for each separate set of a plurality of separate sets of one or more biological cells (72), and wherein each separate set is exposed to a different compound or to a different concentration of the same compound;
wherein the processor (96) is further configured to determine an effect, if any, of each different compound or concentration on the aspect of the secondary peaks (150a, 150b); and
to predict a degree of cardiotoxicity or neurotoxicity of each compound or concentration based on the aspect of the secondary peaks, and a spacing or amplitude regularity or irregularity of the primary peaks or a number, frequency, or period of primary peaks that are smaller peaks having an amplitude below a predefined threshold.

## Patentansprüche

1. Verfahren zur Analyse, wobei das Verfahren Folgendes umfasst:
Detektieren (54) von Fluoreszenz (91), die einen oszillierenden Ionenfluss darstellt, der einer oder mehreren biologischen Zellen (72) zugeordnet ist, um eine Reihe von Datenpunkten (120) zu erzeugen, die ein Oszillationsmuster (110) beschreiben, wobei das Oszillationsmuster (110) eine Reihe von Ereignissen (138) umfasst, wobei das Oszillationsmuster (110) eine vordefinierte Auslöseschwelle (144) zweimal für jedes Ereignis (138) überschreitet, und wobei die Auslöseschwelle(144) relativ zu einer Grundlinie (116) des Oszillationsmusters (110) eingestellt wird;
Identifizieren (62) von Primärpeaks (118) und Sekundärpeaks (150a, 150b) in dem Oszillationsmuster (110), wobei jedes Ereignis (138) einen einzelnen Primärpeak (118) einschließt und wobei jeder der Sekundärpeaks (150a, 150b) in einem Ereignis der Reihe von Ereignissen (138) eingeschlossen ist, und wobei jeder Sekundärpeak ein beliebiger Peak ist, der dem Primärpeak innerhalb eines Ereignisses folgt; und
Bestimmen eines Aspekts der Sekundärpeaks (150a, 150b);
wobei das Detektieren, Identifizieren und Bestimmen für jeden separaten Satz einer Vielzahl von separaten Sätzen einer oder mehrerer biologischer Zellen (72) durchgeführt wird, und wobei jeder separate Satz einer anderen Verbindung oder einer anderen Konzentration derselben Verbindung ausgesetzt wird;
weiter umfassend das Bestimmen einer etwaigen Wirkung jeder unterschiedlichen Verbindung oder Konzentration auf den Aspekt der Sekundärpeaks (150a, 150b); und
Vorhersagen eines Grades an Kardiotoxizität oder Neurotoxizität jeder Verbindung oder Konzentration auf der Grundlage des Aspekts der Sekundärpeaks und eines Abstands oder einer Amplitudenregelmäßigkeit oder -unregelmäßigkeit der Primärpeaks oder einer Anzahl, Frequenz oder Periode von Primärpeaks, die kleinere Peaks mit einer Amplitude unterhalb eines vordefinierten Schwellenwerts sind.

2. Verfahren nach Anspruch 1, wobei das Bestimmen eines Aspekts der Sekundärpeaks (150a, 150b) das Bestimmen einer Anzahl, Frequenz oder Periode der Sekundärpeaks (150a, 150b) einschließt.

3. Verfahren nach Anspruch 1 oder 2,
weiter umfassend das Bestimmen einer Abstandsregelmäßigkeit/- unregelmäßigkeit der Primärpeaks (118) und/oder einer Amplitudenregelmäßigkeit/- unregelmäßigkeit der Primärpeaks (118); oder
weiter umfassend das Vergleichen einer Amplitude (194) jedes Primärpeaks (118) mit einem vordefinierten Schwellenwert, um kleinere Peaks, falls vorhanden, der Primärpeaks zu spezifizieren.

4. Verfahren nach Anspruch 1, 2 oder 3, weiter umfassend das Markieren (52) der einen oder mehreren biologischen Zellen (72) mit einem Calciumindikator, wobei die Fluoreszenz (91) von dem Calciumindikator emittiert wird.

5. System (70), umfassend:
einen optischen Sensor (88), der so konfiguriert ist, dass er Fluoreszenz (91) detektiert, die einen oszillierenden Ionenfluss darstellt, der einer oder mehreren biologischen Zellen (72) zugeordnet ist, um eine Reihe von Datenpunkten (120) zu erzeugen, die ein Oszillationsmuster (110) beschreiben; und
einen Prozessor (96), der so konfiguriert (1) ist, dass er Primärpeaks (118) und Sekundärpeaks (150a, 150b) in dem Oszillationsmuster (110) identifiziert, und (2) einen Aspekt der Sekundärpeaks (150a, 150b) für ein Oszillationsmuster (110) bestimmt, umfassend
eine Reihe von Ereignissen (138), wobei
das Oszillationsmuster (110) eine vordefinierte Auslöseschwelle (144) zweimal für jedes Ereignis (138) überschreitet, und wobei die Auslöseschwelle(144) relativ zu einer Grundlinie (116) des Oszillationsmusters (110) eingestellt wird;
wobei jedes Ereignis (138) einen einzelnen Primärpeak (118) einschließt und wobei jeder der Sekundärpeaks (150a, 150b) in einem Ereignis der Reihe von Ereignissen (138) eingeschlossen ist, wobei jeder Sekundärpeak ein beliebiger Peak ist, der dem Primärpeak innerhalb eines Ereignisses folgt; und
wobei das Detektieren, Identifizieren und Bestimmen für jeden separaten Satz einer Vielzahl von separaten Sätzen einer oder mehrerer biologischer Zellen (72) durchgeführt wird, und wobei jeder separate Satz einer anderen Verbindung oder einer anderen Konzentration derselben Verbindung ausgesetzt wird;
wobei der Prozessor (96) weiter so konfiguriert ist, dass er eine etwaige Wirkung jeder unterschiedlichen Verbindung oder Konzentration auf den Aspekt der Sekundärpeaks (150a, 150b) bestimmt; und
einen Grad der Kardiotoxizität oder Neurotoxizität jeder Verbindung oder Konzentration auf der Grundlage des Aspekts der Sekundärpeaks und eines Abstands oder einer Amplitudenregelmäßigkeit oder -unregelmäßigkeit der Primärpeaks oder einer Anzahl, Frequenz oder Periode von Primärpeaks, die kleinere Peaks aufweisend eine Amplitude unterhalb eines vordefinierten Schwellenwerts vorhersagt.

## Revendications

1. Procédé d'analyse, le procédé comprenant :
une détection (54) d'une fluorescence (91) représentant un flux d'ions oscillant associé à une ou plusieurs cellules biologiques (72), pour produire une série de points de données (120) décrivant un modèle d'oscillation (110), dans lequel le modèle d'oscillation (110) comprend une série d'événements (138), dans lequel le modèle d'oscillation (110) traverse deux fois un niveau de déclenchement prédéfini (144) pour chaque événement (138), et dans lequel le niveau de déclenchement (144) est défini par rapport à une ligne de base (116) du modèle d'oscillation (110) ;
une identification (62) de pics primaires (118) et de pics secondaires (150a, 150b) dans le modèle d'oscillation (110), dans lequel chaque événement (138) inclut un seul pic primaire (118) et dans lequel chacun des pics secondaires (150a, 150b) est inclus dans un événement de la série d'événements (138), et dans lequel chaque pic secondaire est n'importe quel pic suivant le pic primaire au sein d'un événement ; et
une détermination d'un aspect des pics secondaires (150a, 150b) ; dans lequel la détection, l'identification, et la détermination sont mises en œuvre pour chaque ensemble distinct d'une pluralité d'ensembles distincts d'une ou plusieurs cellules biologiques (72), et dans lequel chaque ensemble distinct est exposé à un composé différent ou à une concentration différente du même composé ;
comprenant en outre une détermination d'un effet, le cas échéant, de chaque composé ou concentration différent(e) sur l'aspect des pics secondaires (150a, 150b) ; et
une prédiction d'un degré de cardiotoxicité ou de neurotoxicité de chaque composé ou concentration sur la base de l'aspect des pics secondaires, et d'une régularité ou d'une irrégularité d'espacement ou d'amplitude des pics primaires ou d'un nombre, d'une fréquence, ou d'une période de pics primaires qui sont des pics plus petits présentant une amplitude inférieure à un seuil prédéfini.

2. Procédé selon la revendication 1, dans lequel la détermination d'un aspect des pics secondaires (150a, 150b) inclut une détermination d'un nombre, d'une fréquence, ou d'une période des pics secondaires (150a, 150b).

3. Procédé selon la revendication 1 ou la revendication 2,
comprenant en outre une détermination d'une régularité/irrégularité d'espacement des pics primaires (118) et/ou, d'une régularité/irrégularité d'amplitude des pics primaires (118) ; ou comprenant en outre une comparaison d'une amplitude (194) de chaque pic primaire (118) à un seuil prédéfini pour énumérer des pics plus petits, le cas échéant, des pics primaires.

4. Procédé selon la revendication 1, la revendication 2 ou la revendication 3, comprenant en outre un marquage (52) de la ou des cellules biologiques (72) avec un indicateur de calcium, dans lequel la fluorescence (91) est émise par l'indicateur de calcium.

5. Système (70), comprenant :
un capteur optique (88) configuré pour détecter une fluorescence (91) représentant un flux d'ions oscillant associé à une ou plusieurs cellules biologiques (72), pour produire une série de points de données (120) décrivant un modèle d'oscillation (110) ; et
un processeur (96) configuré pour (1) identifier des pics primaires (118) et des pics secondaires (150a, 150b) dans le modèle d'oscillation (110), et (2) pour déterminer un aspect des pics secondaires (150a, 150b) pour un modèle d'oscillation (110) comprenant
une série d'événements (138), dans lequel le modèle d'oscillation (110) traverse deux fois un niveau de déclenchement prédéfini (144) pour chaque événement (138), et dans lequel le niveau de déclenchement (144) est défini par rapport à une ligne de base (116) du modèle d'oscillation (110) ;
dans lequel chaque événement (138) inclut un seul pic primaire (118) et dans lequel chacun des pics secondaires (150a, 150b) est inclus dans un événement de la série d'événements (138), dans lequel chaque pic secondaire est n'importe quel pic suivant le pic primaire au sein d'un événement ; et
dans lequel la détection, l'identification, et la détermination sont mises en œuvre pour chaque ensemble distinct d'une pluralité d'ensembles distincts d'une ou plusieurs cellules biologiques (72), et dans lequel chaque ensemble distinct est exposé à un composé différent ou à une concentration différente du même composé ;
dans lequel le processeur (96) est en outre configuré pour déterminer un effet, le cas échéant, de chaque composé ou concentration différent(e) sur l'aspect des pics secondaires (150a, 150b) ; et
pour prédire un degré de cardiotoxicité ou de neurotoxicité de chaque composé ou concentration sur la base de l'aspect des pics secondaires, et d'une régularité ou d'une irrégularité d'espacement ou d'amplitude des pics primaires ou d'un nombre, d'une fréquence, ou d'une période de pics primaires qui sont des pics plus petits présentant une amplitude inférieure à un seuil prédéfini.
